(19) Europäisches Patentamt
European Patent Office
Office européen des brevets

(11) Numéro de publication: **0 310 484 A1**

# (12) DEMANDE DE BREVET EUROPEEN

(21) Numéro de dépôt: **88402416.7**

(22) Date de dépôt: **26.09.88**

(51) Int. Cl.$^{4}$: **C 07 C 149/00**
C 07 C 69/00, C 07 C 87/24,
A 61 K 31/10

(30) Priorité: **28.09.87 FR 8713357**

(43) Date de publication de la demande:
**05.04.89 Bulletin 89/14**

(84) Etats contractants désignés:
**AT BE CH DE ES FR GB GR IT LI LU NL SE**

(71) Demandeur: **RHONE-POULENC SANTE
20, avenue Raymond Aron
F-92160 Antony (FR)**

(72) Inventeur: **Malleron, Jean-Luc
2 Allée Renoir
F-91460 Marcoussis (FR)**

**Ponsinet, Gérard
7 Rue du Grand Champ
F-94370 Sucy-en-Brie (FR)**

**Roussel, Gérard
20ter Rue des Carrières
F-91450 Soisy-sur-Seine (FR)**

(74) Mandataire: **Savina, Jacques et al
RHONE-POULENC INTERSERVICES Service Brevets Chimie 25, quai Paul Doumer
F-92408 Courbevoie Cédex (FR)**

Revendications pour les Etats contractants suivants: ES + GR.

(54) **Dérivés d'alcadienes, leurs préparations, les médicaments les contenant et produits intermédiaires.**

(57) Composés de formule:

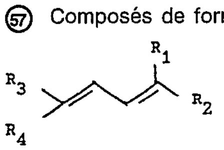

(I)

dans laquelle $R_1$ = hydroxy ou acétoxy, $R_2$ = hydrogène, carboxy, alcoxycarbonyle, phényle ou benzoyle et
- soit $R_3$ = alkylthio ou alcoxy et $R_4$ = naphtoyle ou benzoyle éventuellement substitué
- soit $R_3$ = alcoxycarbonyle , cycloalkyloxycarbonyle ou cyano et $R_4$ = alkyle, naphtyle, phényle éventuellement substitué, alkylthio, naphtylméthanethio, benzylthio éventuellement substitué, phénylthio éventuellement substitué, naphtylthio, phénéthylthio ou allylthio,
- soit $R_3$ et $R_4$ forment avec l'atome de carbone auquel ils sont rattachés un cycle

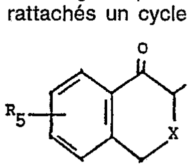

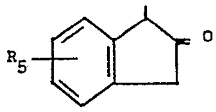

dans lesquelles $R_5$ = hydrogène ou alcoxy et X = méthylène ou S.

Procédés de préparation de ces composés, produits intermédiaires pour leur préparation et médicaments les contenant.

EP 0 310 484 A1

**Description**

**DERIVES D'ALCADIENES, LEURS PREPARATIONS, LES MEDICAMENTS LES CONTENANT ET PRODUITS INTERMEDIAIRES**

La présente invention concerne des dérivés de formule :

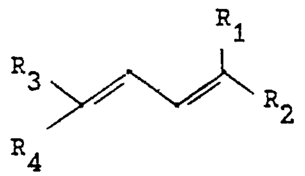

(I)

leurs procédés de préparation, les médicaments les contenant et les produits intermédiaires utiles à leur préparation.

Dans la formule (I), $R_1$ représente un radical hydroxy ou acétoxy, $R_2$ représente un atome d'hydrogène, un radical carboxy, alcoxycarbonyle, phényle ou benzoyle et

- soit $R_3$ représente un radical alkylthio ou alcoxy et $R_4$ représente un radical naphtoyle, benzoyle ou benzoyle substitué par un ou plusieurs atomes d'halogène, un ou plusieurs radicaux alkyle, alcoxy, phényle, phénoxy, pipéridino, diméthylamino ou hydroxy ou en positions -3 et -4 par un radical isopropylènedioxy,
- soit $R_3$ représente un radical alcoxycarbonyle, cycloalkyloxycarbonyle dont la partie cycloalkyle comporte 3 à 6 atomes de carbone ou cyano et $R_4$ représente un radical alkyle (1-8C), naphtyle, phényle éventuellement substitué (par un radical phénoxy, phényle, naphtyle ou benzoyle), alkylthio, naphtylméthanethio, benzylthio éventuellement substitué (par un ou plusieurs radicaux alkyle ou un radical trifluorométhyle, phényle ou phénoxy), phénylthio éventuellement substitué (par un atome d'halogène ou un radical alcoxy), naphtylthio, phénéthylthio ou allylthio,
- soit $R_3$ et $R_4$ forment ensemble avec l'atome de carbone auquel ils sont rattachés un des cycles de formules :

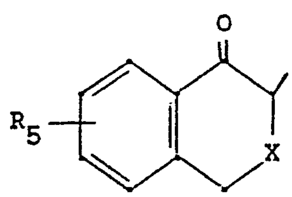

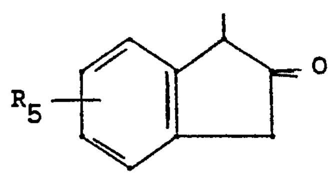

dans lesquelles $R_5$ représente un atome d'hydrogène ou un radical alcoxy et X représente un radical méthylène ou un atome de soufre, étant entendu que, lorsque $R_1$ représente un radical acétoxy, $R_4$ ne peut pas représenter un radical benzoyle substitué par un ou plusieurs radicaux hydroxy, que, sauf mention contraire, dans les définitions qui précèdent et celles qui seront citées ci-après, les radicaux alkyle et alcoxy, et les portions alkyle et alcoxy contiennent 1 à 4 atomes de carbone en chaîne droite ou ramifiée et que l'invention concerne également les formes tautomères des produits cités lorsque $R_1$ représente un radical hydroxy.

Selon l'invention, les produits de formule (I) dans laquelle $R_1$ représente le radical hydroxy, $R_2$ représente un atome d'hydrogène ou un radical alcoxycarbonyle, phényle ou benzoyle et les autres symboles sont définis comme précédemment peuvent être préparés par hydrolyse acide des énamines de formule :

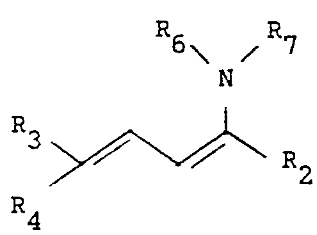

(II)

dans laquelle $R_2$ représente un atome d'hydrogène ou un radical alcoxycarbonyle, phényle ou benzoyle, $R_6$ et $R_7$ représentent des radicaux alkyle ou $R_6$ et $R_7$ forment ensemble avec l'atome d'azote auquel ils sont rattachés un hétérocycle tel que le cycle pipéridine ou le cycle morpholine, $R_3$ et $R_4$ ont la même signification que dans la formule (I).

Il est préférable d'effectuer cette hydrolyse en présence de 1 à 5 équivalents d'un acide minéral 1N à 12N, éventuellement en présence d'un solvant miscible à l'eau, à une température variant de 20° C à 100° C. Comme acide minéral, on peut citer les acides chlorhydrique, bromhydrique et sulfurique et comme solvant, les alcools (méthanol, éthanol, propanol, isopropanol), le tétrahydrofuranne ou le dioxanne.

Ces énamines, excepté le diméthylamino-2 cyano-5 phényl-5 pentadiène-2,4 oate d'éthyle, sont nouvelles et font partie de l'invention.

Les énamines de formule (II) peuvent être préparées par action d'un sel de vinamidinium de formule :

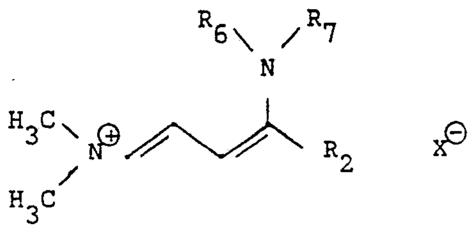

(III)

dans laquelle $R_2$, $R_6$ et $R_7$ ont la même signification que dans la formule (II) et X représente $BF_4$, Cl, $ClO_4$, Br, sur un composé à méthylène activé $R_3$-$CH_2$-$R_4$ pour lequel $R_3$ et $R_4$ ont la même signification que dans la formule (II).

Cette réaction s'effectue en présence d'une base parmi lesquelles on peut citer le méthylate, l'éthylate ou le tertiobutylate de sodium ou de potassium, dans l'alcool correspondant à une température comprise entre 20° C et la température d'ébullition du solvant. On peut également utiliser comme base un dérivé lithié tel que le méthyllithium, le butyllithium, le diisopropylamidure de lithium dans un solvant inerte tel que le tétrahydrofuranne ou l'oxyde de diéthyle à une température comprise entre -78° C et la température d'ébullition du solvant.

Les sels de vinamidinium de formule (III) peuvent être préparés par action d'une amine secondaire $HNR_6R_7$ pour laquelle $R_6$ et $R_7$ ont la même signification que dans la formule (II), sur les produits de formule :

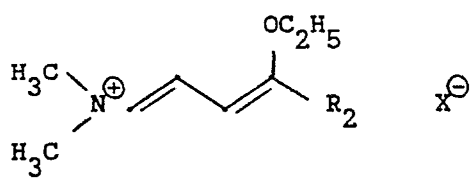

(IV)

dans laquelle $R_2$ et X ont la même signification que dans la formule (III).

La réaction est effectuée de préférence dans un solvant chloré tel que le chloroforme, le chlorure de méthylène ou le dichloro-1,2 éthane, à une température comprise entre 0° C et la température d'ébullition du solvant.

Les composés de formule (IV) peuvent être obtenus par application ou adaptation de la méthode décrite par R. GOMPPER et coll., Angew. Chem. Int. Ed. Eng., 17, 760-3 (1978). Cette méthode consiste à faire réagir un sel d'alkyloxonium comme le tétrafluoroborate de triéthyloxonium, sur une énamine de formule :

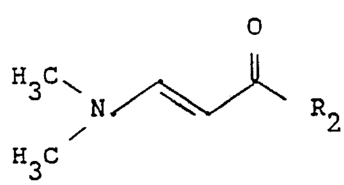

(V)

dans laquelle $R_2$ a la même signification que dans la formule (IV).

La réaction est, de préférence, effectuée dans un solvant chloré tel que le dichlorométhane ou le chloroforme, à une température comprise entre 20°C et la température d'ébullition du solvant.

Les composés de formule (V) peuvent être obtenus par application ou adaptation des méthodes décrites par R.F. ABDULLA et coll. Tetrahedron 35,1675-1734 (1979). Ces méthodes consistent à faire réagir un dialkylacétal de N,N-diméthylformamide avec un composé $CH_3$-CO-$R_2$ pour lequel $R_2$ a la même signification que dans la formule (V).

De nombreux composés de formule $R_3$-$CH_2$-$R_4$ sont connus. Parmi ceux-ci, on peut citer l'α-méthoxyacétophénone (R.B. MOFFET et al, Org. Synth., 3, 562, 1955), l'α-tétralone (Beil 7, 370), le méthoxy-6 oxo-1 tétrahydro-1,2,3,4 naphtalène (Beil 9(2), 889), le propionitrile (Beil 2, 245), le décanenitrile (Beil 2,356), le benzylcyanure (Beil 9, 441), l'indanone-2 (Beil 7, 363), le phénylacétate d'éthyle (Beil 9, 434), le biphénylacétonitrile-4 (brevet US 3 780 065), le phénoxy-3 benzylcyanure (D. MATTHIES et coll., Arch. Pharm. 316, 598-608, 1938), le benzoyl-3 benzylcyanure (A. ALLAIS et coll., J. Med. Chem. Chim. Ther, 9(4), 381-389, 1974).

Les composés de formule $R_3$-$CH_2$-$R_4$ nouveaux peuvent être obtenus par application ou adaptation des méthodes décrites pour les composés connus et des méthodes décrites ci-dessous et dans les exemples.

Les composés de formule $R_3$-$CH_2$-$R_4$ dans laquelle $R_3$ représente un radical alcoxycarbonyle ou cyano et $R_4$ représente un radical phénylthio éventuellement substitué par un atome d'halogène ou un radical alcoxy, un radical naphtylthio, benzylthio, éventuellement substitué par un ou plusieurs radicaux alkyle ou un radical trifluorométhyle, phényle ou phénoxy, un radical phénéthylthio, allylthio, alkylthio ou naphtylméthanethio peuvent être obtenus par condensation d'un thiol ou d'un thiolate $R_8$-S-$R_9$ pour lequel $R_8$ représente un radical phényle éventuellement substitué par un atome d'halogène ou un radical alcoxy, un radical naphtyle, benzyle éventuellement substitué par un ou plusieurs radicaux alkyle ou un radical trifluorométhyle, phényle ou phénoxy, un radical allyle, alkyle, naphtylméthylène ou phénéthyle et $R_9$ représente le sodium, le potassium, le lithium ou un atome d'hydrogène sur un composé de formule X-$CH_2$-$R_3$ dans laquelle X représente un atome d'halogène et $R_3$ a la même signification que précédemment.

Lorsque $R_9$ représente le sodium, le potassium ou le lithium, la réaction s'effectue de préférence dans un solvant tel qu'un alcool (méthanol, éthanol, propanol, isopropanol), le diméthylformamide, le tétrahydrofuranne ou l'oxyde de diéthyle, à une température comprise entre 0°C et la température d'ébullition du solvant. Les thiolates $R_8$-S-$R_9$ peuvent être obtenus par action d'une base telle que le méthylate, l'éthylate ou le tertiobutylate de sodium ou de potassium ou d'une base lithiée telle que le butyllithium sur le sulfure correspondant.

Lorsque $R_9$ représente l'atome d'hydrogène, la réaction s'effectue de préférence dans un solvant tel que le méthanol, l'éthanol, le propanol, l'isopropanol, le diméthylformamide, le tétrahydrofuranne, l'oxyde de diéthyle ou un solvant chloré (chlorure de méthylène, dichloro-1,2 éthane), en présence d'une base tertiaire telle qu'une trialcoylamine, le diaza-1,8 bicyclo [5.4.0.)undécène-7 ou le diaza-1,5 bicyclo[4.3.0.]nonène-5.

Les thiols pour lesquels $R_8$ représente un radical benzyle substitué par un radical alkyle, trifluorométhyle, phényle ou phénoxy peuvent être obtenus à partir des halogénures correspondants $R_8$ -X pour lesquels $R_8$ a la même signification que précédemment et X représente un atome d'halogène par action de sulfhydrate de sodium par des procédés connus en soi tels que ceux décrits par MARCH, Advanced Organic Chemistry, 1977, p. 374 (second edition). Les halogénures $R_8$-X peuvent être obtenus par application ou adaptation des méthodes décrites par J. ASHBY et coll., Carcinogenesis, 2(1), 33-38, 1981, D. MATTHIES et coll., Arch. Pharm.,316, 598-608, 1983, J.L. RIDEOUT et coll., J. Med. Chem. 1489, 1983 et dans Beil. 5, 364, 373 et 384.

Les composés de formule $R_3$-$CH_2$-$R_4$ dans laquelle $R_3$ représente un radical alcoxycarbonyle et $R_4$ représente un radical benzylthio éventuellement substitué par un radical phényle, phénoxy, alkyle ou trifluorométhyle peuvent être obtenus par condensation du mercaptoacétate d'éthyle sur un composé de formule X-$R_{10}$ dans laquelle X représente un atome d'halogène et $R_{10}$ représente un radical benzyle éventuellement substitué par un radical phényle, phénoxy, alkyle ou trifluorométhyle.

La réaction s'effectue de préférence dans un solvant tel qu'un alcool (méthanol, éthanol, propanol, isopropanol), le diméthylformamide, le tétrahydrofuranne ou l'oxyde de diéthyle, en présence d'une base telle que le méthylate, l'éthylate ou le tertiobutylate de sodium ou de potassium ou d'une base lithiée telle que le butyllithium sur le mercaptoacétate d'éthyle à une température comprise entre 0°C et la température d'ébullition du solvant.

Les composés de formule $R_3$-$CH_2$-$R_4$ dans laquelle $R_3$ représente un radical alcoxycarbonyle ou cycloalkyloxycarbonyle et $R_4$ représente un radical alkyle (1-8C), naphtyle, phényle éventuellement substitué par un radical phénoxy, phényle, naphtyle ou benzoyle, un radical alkylthio, naphtylméthanethio, benzylthio éventuellement substitué par un ou plusieurs radicaux alkyle ou un radical trifluorométhyle, phényle ou phénoxy, un radical phénylthio éventuellement substitué par un atome d'halogène ou un radical alcoxy, un radical naphthylthio, phénéthylthio ou allylthio peuvent être préparés par estérification des acides correspondants par des procédés connus en soi tels que ceux décrits par MARCH, Advanced Organic Chemistry, 1977, p. 363-367 (second edition).

De préférence, l'estérification est effectuée au moyen d'un alcool en présence d'un acide minéral.

Les acides pour lesquels $R_4$ représente un radical phényle éventuellement substitué par un radical phénoxy, phényle, naphtyle ou benzoyle, peuvent être obtenus par application ou adaptation des méthodes décrites par D. MATTHIES et coll., Arch. Pharm., 316, 598-608, 1983 ; A. ALLAIS et coll., Eur. J. Med. Chem. Chim. Ther., 9(4), 381-389, 1974 et R.M. SHAFIF et coll., J. Pharm. Sci, 952-955, 1969.

Les acides pour lesquels $R_3$ représente un radical carboxy et $R_4$ représente un radical alkylthio, naphtylméthanethio, benzylthio éventuellement substitué par un ou plusieurs radicaux alkyle ou un radical trifluorométhyle, phényle ou phénoxy, un radical phénylthio éventuellement substitué par un atome d'halogène ou un radical alcoxy, un radical naphtylthio, phénéthylthio ou allylthio peuvent être préparés par hydrolyse des esters éthyliques ou des dérivés cyanés correspondants décrits précédemment par des procédés connus en soi tels que ceux décrits par MARCH, Advanced Organic Chemistry, 1977, p. 349-354 (second edition).

Les composés de formule $R_3$-$CH_2$-$R_4$ dans laquelle $R_3$ représente un radical alkylthio et $R_4$ représente un radical naphtoyle, benzoyle ou benzoyle substitué par un ou plusieurs atomes d'halogène, un ou plusieurs radicaux alkyle, alcoxy, phényle, phénoxy, hydroxy, pipéridino, diméthylamino ou en positions -3 et -4 par un radical isopropylènedioxy peuvent être préparés par action des composés $R_{11}$ -SNa pour lesquels $R_{11}$ représente un radical alkyle, sur un halogénure de formule $R_4$-$CH_2$-X dans laquelle $R_4$ a la même signification que précédemment et X représente un atome d'halogène. Cette réaction s'effectue de préférence dans un alcool tel que le méthanol ou l'éthanol à une température comprise entre 20°C et 60°C.

Le chlorure de formule $R_4$-$CH_2Cl$ dans laquelle $R_4$ représente un radical benzoyle substitué par un radical pipéridine peut être préparé par action de chlorure de titane III sur le dérivé dibromé $R_4$-CO-CH-$Br_2$ pour lequel $R_4$ a la même signification que précédemment, en présence d'un acide minéral tel que l'acide chlorhydrique à une température d'environ 100°C.

Le composé de formule $R_4$-CO-CH-$Br_2$ peut être obtenu par action d'un mélange brome-acide bromhydrique sur le dérivé cétonique correspondant, de préférence à 20°C.

Les autres nouveaux halogénures de formule $R_4$-$CH_2$-X peuvent être préparés par halogénation des composés $R_4$-$CH_3$ correspondants par des méthodes connues en soi telles que celles décrites par MARCH, Advanced Organic Chemistry, 1977, p. 537-39 (second edition) ou par adaptation des méthodes de préparation des halogénures connus.

Les composés $R_{11}$-SNa peuvent être obtenus par action d'une base telle que le méthylate ou l'éthylate de sodium sur le thiol correspondant.

Les composés de formule $R_3$-$CH_2$-$R_4$ dans laquelle $R_3$ représente un radical alcoxy et $R_4$ représente un radical naphtoyle, benzoyle ou benzoyle substitué par un ou plusieurs atomes d'halogène ou un ou plusieurs radicaux phényle, phénoxy, hydroxy, pipéridino, diméthylamino, ou en positions -3 et -4 par un radical isopropylènedioxy peuvent être obtenus par application ou adaptation de la méthode décrite par R,B. MOFFET et al., Org. Synth., 3, 562 (1955).

Les composés de formule :

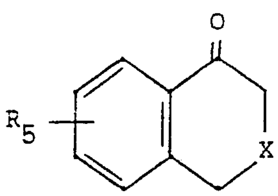

dans laquelle $R_5$ représente un atome d'hydrogène ou un radical alcoxy et X représente un atome de soufre peuvent être obtenus en chauffant l'acide benzylthioacétique éventuellement substitué par un radical alcoxy dans un solvant tel que le nitrobenzène, en présence d'un acide tel que l'acide polyphosphorique à une température comprise entre 50°C et 180°C.

Selon l'invention, les produits de formule (I) dans laquelle $R_1$ représente le radical hydroxy, $R_2$ représente un radical alcoxycarbonyle et les autres symboles sont définis comme précédemment peuvent également être préparés par hydrolyse acide des énamines de formule :

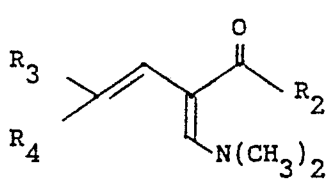

(VI)

dans laquelle $R_2$ représente un radical alcoxycarbonyle, $R_3$ et $R_4$ ont la même signification que dans la formule (I).

Cette hydrolyse peut s'effectuer dans les mêmes conditions opératoires que l'hydrolyse des énamines de formule (II).

Les énamines de formule (VI) peuvent être obtenues par action d'un dialkylacétal de N,N-diméthylformamide sur un acide correspondant de formule (I) dans laquelle $R_2$ représente le radical carboxy, $R_1$ représente le radical hydroxy, $R_3$ et $R_4$ sont définis comme précédemment. On opère de préférence dans un solvant chloré tel que le chloroforme ou le dichlorométhane à une température voisine de 20°C.

Les énamines de formule (VI) sont nouvelles et font partie de l'invention.

Selon l'invention, les produits de formule (I) dans laquelle $R_1$ représente un radical hydroxy, $R_2$ représente le radical carboxy et les autres symboles sont définis comme précédemment, peuvent être préparés par saponification de l'ester énol correspondant de formule :

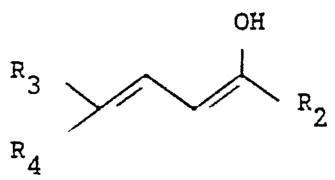

(VII)

dans laquelle $R_2$ représente un radical alcoxycarbonyle et $R_3$, $R_4$ sont définis comme précédemment.

De préférence, cette réaction s'effectue au moyen de 2 à 10 équivalents de soude ou de potasse 1N à 4N éventuellement en présence d'un solvant inerte miscible à l'eau tel que le méthanol, l'éthanol, le propanol, l'isopropanol ou le tétrahydrofurane, à une température comprise entre 20°C et 50°C.

Selon l'invention, les produits de formule (I) dans laquelle $R_1$ représente un radical acétoxy et les autres symboles sont définis comme précédemment, étant entendu que $R_3$ ne peut pas représenter un radical benzoyle substitué par un ou plusieurs radicaux hydroxy, peuvent être obtenus par action du chlorure d'acétyle sur l'énol correspondant de formule (I) dans laquelle $R_1$ représente le radical hydroxy. Cette réaction s'effectue de préférence dans un solvant inerte tel que le tétrahydrofuranne en présence d'un accepteur d'acide tel que la triéthylamine à une température d'environ 20°C.

Les mélanges réactionnels obtenus par les divers procédés décrits précédemment sont traités suivant des méthodes classiques physiques (évaporation, extraction, distillation, cristallisation, chromatographie) ou chimiques.

Les composés de formule (I) présentent des propriétés pharmacologiques intéressantes. Ces composés inhibent la 5-lipoxygénase et sont donc utiles comme antiinflammatoires, comme agents protecteurs notamment sur le tractus gastrointestinal et pour le traitement de l'asthme, des maladies allergiques, du psoriasis, de l'arthrite rhumatoïde et des fibroses notamment des fibroses hépathiques.

L'inhibition de la 5-lipoxygénase a été déterminée sur des cellules RBL-1 selon les méthodes de M.M. STEINHOFF et coll., B.B.B., 618, 28-34, 1980 et B.A. JAKSCHIK et coll., J. Biol. Chem., 257, 5346, 1982. Dans ce test, la $CI_{50}$(M) des composés de formule (I) est comprise entre $10^{-5}$ et $10^{-7}$.

Ces composés sont également actifs sur cellules PMN selon la méthode de H. SAFAYHI et coll., Biochem. Pharmacol., 34(15), 2691-4, 1985. Dans ce test, la $CI_{50}$(M) des composés de formule (I) est comprise entre $10^{-5}$ et $10^{-7}$.

Les produits de formule (I) présentent une faible toxicité. Leur $DL_{50}$ est supérieure à 100 mg/kg par voie orale chez la souris.

Sont particulièrement intéressants:

- les composés de formule (I) dans laquelle
- soit $R_1$ représente un radical hydroxy, $R_2$ représente un radical alcoxycarbonyle, $R_3$ représente un radical alkylthio et $R_4$ représente un radical benzoyle substitué par un atome d'halogène, un radical pipéridino, deux radicaux hydroxy ou en positions -3 et -4 par un radical isopropylènedioxy ;
- soit $R_1$ représente un radical hydroxy ou acétoxy, $R_2$ représente un atome d'hydrogène, un radical carboxy, alcoxycarbonyle ou phényle, $R_3$ représente un radical alcoxycarbonyle et $R_4$ représente un radical alkylthio ou benzylthio éventuellement substitué par un radical alcoxy
- et les formes tautomères de ces produits lorsque $R_1$ représente un radical hydroxy.

Sont d'un intérêt particulier, les produits suivants :

- acide hydroxy-2 phénylthio-5 éthoxycarbonyle-5 pentadiène-2,4 oïque,
- (chloro-4 phényl)-6 hydroxy-2 méthylthio-5 oxo-6 hexadiène-2,4 oate d'éthyle,
- hydroxy-2 méthylthio-5 oxo-6 (pipéridino-4 phényl)-6 hexadiène-2,4 oate d'éthyle,
- hydroxy-2 (méthoxy-4 phénylthio)-5 hexadiène-2,4 dioacte de diéthyle,
- benzylthio-5 hydroxy-2 hexadiène-2,4 dioate de diéthyle
- hydroxy-5 phénylthio-2 pentadiène-,2,4 oate d'éthyle,
- acétoxy-2 phénylthio-5 hexadiène-,2,4 dioate de diéthyle
- oxo-5 phényl-5 phénylthio-2 pentène-2 oate d'éthyle,
- hydroxy-2 (isopropylènedioxy-3,4 phényl)-6 méthylthio-5 oxo-6 hexadiène-2,4 oate d'éthyle,
- hydroxy-2 méthoxy-5 oxo-6 phényl-6 hexadiène-2,4 oate d'éthyle,
- hydroxy-2 méthylthio-5 hexadiène-2,4 dioate de diéthyle,
- hydroxy-2 éthylthio-5 hexadiène-2,4 dioate de diéthyle.

Les exemples suivants, donnés à titre non limitatif, montrent comment l'invention peut être mise en pratique.

EXEMPLE 1

A une solution éthanolique de 17 g d'hydroxy-2 phénylthio-5 hexadiène-2,4 dioate de diéthyle, on ajoute, en 10 minutes, en maintenant à une température voisine de 20° C, 211 cm3 d'une solution aqueuse de soude 1N. Le mélange est maintenu à une température voisine de 20° C pendant 2 heures 15 minutes puis est amené à pH 1 à l'aide d'une solution aqueuse d'acide chlorhydrique 1N.

L'éthanol réactionnel est évaporé sous pression réduite (20 mm de mercure ; 2,7 kPa). Après addition de 100 cm3 d'eau, le mélange est extrait par 2 fois 150 cm3 d'acétate d'éthyle. La phase organique est séchée sur sulfate de magnésium anhydre et concentrée à sec à 40° C sous pression réduite (20 mm de mercure ; 2,7 kPa). Le solide ainsi obtenu est recristallisé dans un mélange d'oxyde d'isopropyle et d'acétonitrile (50-55 en volumes) bouillant.

On obtient ainsi 9,5 g d'acide hydroxy-2 phénylthio-5 éthoxycarbonyl-5 pentadiène-2,4 oïque fondant à 172° C.

EXEMPLE 2

A une solution de 60 g de diméthylamino-2 phénylthio-5 hexadiène-2,4 dioate de diéthyle dans 340 cm3 d'éthanol, on ajoute, en 10 minutes, en maintenant la température à environ 40° C, 344 cm3 d'une solution aqueuse d'acide chlorhydrique 1N. La suspension obtenue est refroidie à une température d'environ 20° C et est maintenue 30 minutes à cette température. Le précipité est filtré, lavé par 3 fois 50 cm3 d'eau et séché à 20° C sous pression réduite (20 mm de mercure ; 2,7 kPa) en présence d'anhydride phosphorique. On obtient 54 g d'un solide cristallisé. 13 g de ce solide sont recristallisés dans 30 cm3 d'oxyde d'isopropyle bouillant. On obtient ainsi 10 g d'hydroxy-2 phénylthio-5 hexadiène-2,4 dioate de diéthyle fondant à 98° C.

Le diméthylamino-2 phénylthio-5 hexadiène-2,4 dioate de diéthyle peut être obtenu de la façon suivante :

A une solution de 68,7 g phénylthioacétate d'éthyle et de 100 g de tétrafluoroborate de N-(diméthylamino-3 éthoxycarbonyl-3 propénylidène) N-méthyl méthanaminium dans 120 cm3 d'éthanol, on ajoute goutte à goutte, en 50 minutes et en maintenant la température à environ 20° C, 210 cm3 d'une solution éthanolique 2M d'éthylate de sodium. Le mélange réactionnel est maintenu 2 heures 20 minutes à une température voisine de 20° C puis le solvant est distillé sous pression réduite (20 mm de mercure ; 2,7 kPa) à 40° C. Le résidu est repris par 1000 cm3 d'eau distillée et extrait par 3 fois 300 cm3 d'acétate d'éthyle. Après lavage à l'eau, les extraits organiques sont séchés sur sulfate de magnésium anhydre et concentrés à sec sous pression réduite (20 mm de mercure ; 2,7 kPa). Après recristallisation dans 150 cm3 d'oxyde d'isopropyle bouillant, on obtient 107 g d'un solide fondant à 70° C.

15 g de ce solide sont chromatographiés sur une colonne de 6 cm de diamètre contenant 450 g de silice avec un mélange de cyclohexane et d'acétate d'éthyle (80-20 en volumes) comme éluant. La première fraction de 800 cm3 est éliminée puis on recueille des fractions de 100 cm3. Les fractions 4 à 11 sont réunies et concentrées à sec à 40° C sous pression réduite (20 mm de mercure ; 2,7 kPa). Après recristallisation dans 40 cm3 d'oxyde d'isopropyle bouillant, on obtient 13,2 g de diméthylamino-2 phénylthio-5 hexadiène-2,4 dioate de diéthyle fondant à 76° C.

Le tétrafluoroborate de N-(diméthylamino-3 éthoxycarbonyl-3 propénylidène) N-méthyl méthanaminium peut être obtenu de la façon suivante :

A une solution chlorométhylénique de 1007 g de tétrafluoroborate de N-(éthoxy-3 éthoxycarbonyl-3 propénylidène) N-méthyl méthanaminium, on ajoute, en 1 heure 20 minutes, à une température d'environ 20° C, une solution chlorométhylénique de 233 cm3 de diméthylamine. On coule ensuite lentement dans le milieu réactionnel 2000 cm3 d'éther éthylique anhydre puis sépare par filtration le produit cristallisé et le sèche sous pression réduite (20 mm de mercure ; 2,7 kPa) à une température d'environ 20° C. On obtient ainsi 925 g de tétrafluoroborate de N-(diméthylamino-3 éthoxycarbonyl-3 propénylidène) N-méthyl méthanaminium fondant à 102° C.

Le tétrafluoroborate de N-(éthoxy-3 éthoxycarbonyl-3 propénylidène) N-méthyl méthanaminium peut être obtenu de la façon suivante :

A une solution chlorométhylénique de 862 g de tétrafluoroborate de triéthyloxonium refroidie à 10° C et traversée par un courant d'argon, on ajoute, en 3 heures, 675 g de diméthylamino-4 oxo-2 butène-3 oate d'éthyle en maintenant la température à environ 10° C. Le milieu réactionnel est ensuite agité 5 heures à une température voisine de 20° C. La solution chlorométhylénique de tétrafluoroborate de N-(éthoxy-3 éthoxycarbonyl-3 propénylidène) N-méthyl méthanaminium ainsi obtenue est conservée sous une atmosphère d'argon et est utilisée immédiatement dans les synthèses ultérieures.

Le diméthylamino-4 oxo-2 butène-3 oate d'éthyle peut être obtenu de la façon suivante :

A 2100 g d'acétal diéthylique de N,N-diméthylformamide refroidis à une température voisine de 5° C, on ajoute en 2 heures, 1500 g de pyruvate d'éthyle en maintenant la température à environ 5° C. Le mélange est ensuite agité 3 heures à une température voisine de 20° C. L'éthanol réactionnel est évaporé à sec et le résidu obtenu est battu par 12 litres d'éther diéthylique et 30 g de noir 3S puis filtré sur supercel. Le filtrat est repris par 500 cm3 d'eau, la phase organique est décantée et la phase aqueuse est extraite par 3 fois 2000 cm3 de dichlorométhane. Les phases organiques sont réunies, séchées sur sulfate de sodium, filtrées et concentrées à sec à 40° C sous pression réduite (20 mm de mercure ; 2,7 kPa). L'huile obtenue est purifiée par distillation

($Eb_{0,1mmHg}$ = 146-149°C). On obtient ainsi 695 g de diméthylamino-4 oxo-2 butène-3 oate d'éthyle utilisé à l'état brut dans les synthèses ultérieures.

Le phénylthioacétate d'éthyle peut être obtenu de la façon suivante :

A 1 400 cm3 d'une solution éthanolique 2M d'éthylate de sodium, on ajoute, goutte à goutte, en 10 minutes, à une température voisine de 20°C, 300 g de thiophénol. On introduit ensuite 454 g de bromoacétate d'éthyle en maintenant la température à environ 10°C. Le précipité obtenu est filtré et la solution éthanolique résiduelle est concentrée à sec à 40°C sous pression réduite (20 mm de mercure ; 2,7 kPa). On distille et obtient 352 g de phénylthioacétate d'éthyle dont le point d'ébullition est de 125°C sous 0,75 mm de mercure (0,1 kPa).

EXEMPLE 3

A une solution de 21 g de diméthylamino-2 (oxo-1 tétrahydro-1,2,3,4 naphtylidène-2)-4 butène-2 oate d'éthyle dans 150 cm3 d'éthanol maintenue à reflux, on ajoute, en 2 minutes, 70 cm3 d'une solution aqueuse d'acide chlorhydrique 1N. Le mélange est immédiatement refroidi à une température voisine de 20 à 30°C et l'éthanol réactionnel est distillé à 40°C sous pression réduite (20 mm de mercure ; 2,7 kPa). La suspension obtenue est diluée par 50 cm3 d'eau et extraite par 2 fois 50 cm3 d'acétate d'éthyle. Après lavage avec 50 cm3 d'eau et séchage, la phase organique est concentrée à sec à 40°C sous pression réduite (20 mm de mercure ; 2,7 kPa). Après recristallisation du solide obtenu dans un mélange d'oxyde d'isopropyle et d'acétate d'éthyle (90-10 en volumes) bouillant, on obtient 11,2 g d'hydroxy-2 (oxo-1 tétrahydro-1,2,3,4 naphtylidène-2)-4 butène-2 oate d'éthyle fondant à 117°C.

Le diméthylamino-2 (oxo-1 tétrahydro-1,2,3,4 naphtylidène-2)-4 butène-2 oate d'éthyle peut être obtenu de la façon suivante :

A un mélange de 100 g de tétrafluoroborate de N-(diméthylamino-3 éthoxycarbonyl-3 propénylidène) N-méthyl méthanaminium dans 500 cm3 d'éthanol et 210 cm3 d'une solution éthanolique 2M d'éthylate de sodium, on ajoute, goutte à goutte, en 45 minutes, à une température voisine de 15°C, 51 g d'α-tétralone. Le mélange est maintenu pendant 1 heure 45 minutes à une température voisine de 20°C puis est chauffé pendant 4 heures 45 minutes à 70°C. L'éthanol réactionnel est évaporé à sec et l'huile obtenue est battue avec 4 fois 300 cm3 d'éther éthylique. Les extraits éthérés sont concentrés à sec sous pression réduite (20 mm de mercure ; 2,7 kPa). Après recristallisation du solide obtenu dans 60 cm3 d'un mélange d'oxyde d'isopropyle et d'acétate d'éthyle (90-10 en volumes) bouillant on obtient 17,5 g de diméthylamino-2 (oxo-1 tétrahydro-1,2,3,4 naphtylidène-2)-4 butène-2 oate d'éthyle fondant à 84°C.

EXEMPLE 4

A une solution de 13 g de phényl-6 oxo-6 méthylthio-5 diméthylamino-2 hexadiène-2,4 oate d'éthyle dans 100 cm3 d'éthanol, on ajoute, en 10 minutes et en maintenant au reflux, 53 cm3 d'une solution aqueuse 1N d'acide chlorhydrique. Le mélange est immédiatement refroidi et l'éthanol réactionnel est concentré à 40°C sous pression réduite (20 mm de mercure ; 2,7 kPa). La suspension obtenue est diluée par 100 cm3 d'eau distillée et extraite par 3 fois 100 cm3 d'oxyde de diéthyle. Après lavage à l'eau, les extraits organiques sont concentrés à sec à 40°C sous pression réduite (20 mm de mercure ; 2,7 kPa). Après recristallisation du solide obtenu dans 45 cm3 d'oxyde d'isopropyle bouillant, on obtient 10,2 g de phényl-6 oxo-6 méthylthio-5 hydroxy-2 hexadiène-2,4 oate d'éthyle fondant à 86°C.

Le phényl-6 oxo-6 méthylthio-5 diméthylamino-2 hexadiène-2,4 oate d'éthyle peut être obtenu de la façon suivante :

A une solution de 26 g de tétrafluoroborate de N-(diméthylamino-3 éthoxycarbonyl-3 propénylidène) N-méthyl méthaniminium et de 30 g d'α-méthylthioacétophénone dans 100 cm3 d'éthanol maintenue à une température voisine de 25°C, on ajoute, goutte à goutte, en 30 minutes, 54 cm3 d'une solution éthanolique 2M d'éthylate de sodium. Le mélange est maintenu pendant 2 heures 10 minutes à une température voisine de 25°C puis l'éthanol réactionnel est éliminé par distillation à 40°C sous pression réduite (20 mm de mercure ; 2,7 kPa). Après recristallisation du résidu dans 100 cm3 d'oxyde d'isopropyle bouillant, on obtient 19,2 g de phényl-6 oxo-6 méthylthio-5 diméthylamino-2 hexadiène-2,4 oate d'éthyle fondant à 78°C.

L'α-méthylthioacétophénone peut être obtenue de la façon suivante :

A une solution de 199 g d'α-bromoacétophénone dans 500 cm3 d'éthanol, on ajoute, en 1 heure 30 minutes, par petites portions et à une température voisine de 20°C, 70 g de méthanethiolate de sodium. Le mélange est maintenu pendant 2 heures 30 minutes à une température voisine de 20°C. Le solide en suspension est éliminé par filtration et l'éthanol réactionnel est éliminé par évaporation à sec à 40°C sous pression réduite (20 mm de mercure ; 2,7 kPa). Le résidu huileux est repris par 400 cm3 d'oxyde de diéthyle, la phase organique est lavée par 4 fois 200 cm3 d'eau, séchée et concentrée à sec à 40°C sous pression réduite (20 mm de mercure ; 2,7 kPa). Après distillation, on obtient 98 g d'α-méthylthioacétophénone distillant à 100-102°C sous une pression de 0,2 mm de mercure (0,027 kPa).

EXAMPLE 5

A une solution de 21,3 g de diméthylamino-2 cyano-5 phényl-5 pentadiène-2,4 oate d'éthyle dans 27 cm3 d'éthanol maintenue à reflux, on ajoute, en 3 minutes, 87 cm3 d'une solution aqueuse d'acide chlorhydrique 1N. Le mélange est refroidi à une température d'environ 10°C et le précipité obtenu est filtré, lavé par 15 cm3 d'éthanol et séché. On obtient ainsi 12,1 g d'hydroxy-2 cyano-5 phényl-5 pentadiène-2,4 oate d'éthyle fondant à 189°C.

Le diméthylamino-2 cyano-5 phényl-5 pentadiène-2,4 oate d'éthyle peut être préparé de la façon suivante :

On opère comme à l'exemple 2 pour la préparation du diméthylamino-2 phénylthio-5 hexadiène-2,4 dioate de diéthyle à partir de 57,2 g de tétrafluoroborate de N-(diméthylamino-3 éthoxycarbonyl-3 propènylidène) N-méthyl méthanaminium, de 105 cm3 d'une solution éthanolique 2M d'éthylate de sodium et de 23,4 g de cyanure de benzyle. On obtient 36,1 g de diméthylamino-2 cyano-5 phényl-5 pentadiène-2,4 oate d'éthyle fondant à 67°C.

EXEMPLE 6

A une solution agitée de 6,3 g de (diméthylamino méthylène)-3 oxo-2 phénylthio-5 hexène-4 dioate de (6) éthyle et de (1) méthyle dans 70 cm3 d'éthanol, chauffée 15 minutes à 50°C, on ajoute 34 cm3 d'une solution aqueuse d'acide chlorhydrique normal. L'agitation est poursuivie 2 heures à une température voisine de 20°C. La solution est concentrée à sec à 40°C sous pression réduite (20 mm de mercure ; 2,7 kPa). Le résidu est lavé à l'eau puis repris par 100 cm3 de dichlorométhane. La phase organique est séchée sur sulfate de magnésium anhydre, filtrée et concentrée à sec à 40°C sous pression réduite (20 mm de mercure ; 2,7 kPa). L'huile obtenue est recristallisée dans 60 cm3 d'oxyde d'isopropyle bouillant. On obtient ainsi 2,7 g d'hydroxy-2 phénylthio-5 hexadiène-2,4 dioate de (6) éthyle et de (1) méthyle fondant à 112°C.

Le (diméthylamino méthylène)-3 oxo-2 phénylthio-5 hexène-4 dioate de (6) éthyle et de (1) méthyle peut être obtenu de la façon suivante :

8 g d'acide hydroxy-2 phénylthio-5 éthoxycarbonyl-5 pentadiène-2,4 oïque, obtenu selon l'exemple 1, 7,2 cm3 de diméthylacétal de N,N-diméthylformamide et 80 cm3 de dichlorométhane sont agités 2 heures à une température voisine de 20°C. La solution est concentrée à sec à 40°C sous pression réduite (20 mm de mercure ; 2,7 kPa). On obtient un résidu que l'on dissout dans 100 cm3 d'un mélange de cyclohexane et d'acétate d'éthyle (90-10 en volumes). La solution est versée sur 500 g de silice contenus dans une colonne de 5 cm de diamètre. On élue avec un mélange de cyclohexane et d'acétate d'éthyle (90-10 en volumes) et l'éluat correspondant est concentré à sec à 40°C sous pression réduite (20 mm de mercure ; 2,7 kPa). On obtient ainsi 5,6 g de (diméthylamino méthylène)-3 oxo-2 phénylthio-5 hexène-4 dioate de (6) éthyle et de (1) méthyle fondant à 118°C.

EXEMPLE 7

On opère comme à l'exemple 3, à partir de 4,2 g de diméthylamino-2 (méthoxy-6 oxo-1 tétrahydro-1,2,3,4 naphtylidène-2)-4 butène-2 oate d'éthyle, de 19,1 cm3 d'une solution aqueuse d'acide chlorhydrique 1N et de 19 cm3 d'éthanol. Le mélange est chauffé pendant 5 minutes à ébullition puis refroidi à une température voisine de 20°C. Après purification par recristallisation dans 120 cm3 d'éthanol bouillant, on obtient 3,4 g d'hydroxy-2 (méthoxy-6 oxo-1 tétrahydro-1,2,3,4 naphtylidène-2)-4 butène-2 oate d'éthyle fondant à 175°C.

Le diméthylamino-2 (méthoxy-6 oxo-1 tétrahydro-1,2,3,4 naphtylidène-2)-4 butène-2 oate d'éthyle peut être préparé de la manière suivante :

On opère comme à l'exemple 3 pour la préparation du diméthylamino-2 (oxo-1 tétrahydro-1,2,3,4 naphtylidène-2)-4 butène-2 oate d'éthyle, a partir de 14,3 g de tétrafluoroborate de N-(diméthylamino-3 éthoxycarbonyl-3 propénylidène) N-méthyl méthanaminium, de 30 cm3 d'une solution éthanolique 2M d'éthylate de sodium et de 8,8 g de méthoxy-6 oxo-1 tétrahydro-1,2,3,4 naphtalène dans 72 cm3 d'éthanol. Après purification par chromatographie sur colonne de silice avec un mélange de cyclohexane et d'acétate d'éthyle (50-50 en volumes) comme éluant et précipitation dans 150 cm3 d'oxyde d'éthyle, on obtient 7,2 g de diméthylamino-2 (oxo-1 tétrahydro-1,2,3,4 naphtylidène-2)-4 butène-2 oate d'éthyle fondant à 123°C.

EXEMPLE 8

On opère comme à l'exemple 3, à partir de 3,8 g de diméthylamino-2 (oxo-2 dihydro-2,3 indénylidène-1)-4 butène-2 oate d'éthyle, de 20 cm3 d'une solution aqueuse d'acide chlorhydrique 1N et de 20 cm3 d'éthanol. Le mélange est chauffé 10 minutes à une température d'environ 60°C puis est refroidi à environ 20°C.

Après purification par chromatographie sur colonne de silice avec un mélange de cyclohexane et d'acétate d'éthyle (70-30 en volumes) comme éluant et recristallisation dans 52 cm3 d'un mélange de cyclohexane et d'ethanol (90-10 en volumes) bouillant, on obtient 1,5 g d'hydroxy-2 (oxo-2 dihydro-2,3 indénylidène-1)-4 butène-2 oate d'éthyle fondant 137°C.

Le diméthylamino-2 (oxo-2 dihydro-2,3 indénylidène-1)-4 butène-2 oate d'éthyle peut être préparé de la manière suivant :

On opère comme à l'exemple 3 pour la préparation du diméthylamino-2 (oxo-1 tétrahydro-1,2,3,4 naphtylidène-2)-4 butène-2 oate d'éthyle, à partir de 14,3 g de tétrafluoroborate de N-(diméthylamino-3 éthoxycarbonyl-3 propénylidène) N-méthyl méthanaminium, de 31,2 cm3 d'une solution 1,6M de butyllithium dans l'hexane et de 6,6 g d'indanone-2 dans 100 cm3 de tétrahydrofuranne à une température voisine de 0°C. Après purification par chromatographie sur colonne de silice avec un mélange de cyclohexane et d'acétate d'éthyle (50-50 en volumes) comme éluant et recristallisation dans 70 cm3 d'oxyde d'isopropyle bouillant, on obtient 4,9 g de diméthylamino-2 (oxo-2 dihydro-2,3 indénylidène-1)-4 butène-2 oate d'éthyle fondant à 93-95°C.

EXEMPLE 9

On opère comme à l'exemple 3, à partir de 16,8 g de diméthylamino-2 (oxo-1 thia-3 tétrahydro-1,2,3,4 naphtylidène-2)-4 butène-2 oate d'éthyle, de 120 cm3 d'une solution aqueuse d'acide chlorhydrique 1N et de 120 cm3 d'éthanol. Le mélange est agité pendant 15 heures à une température voisine de 20°C. Après purification par chromatographie sur colonne de silice avec du dichlorométhane comme éluant et recristallisation dans 40 cm3 d'isopropanol bouillant, on obtient 1,5 g d'hydroxy-2 (oxo-1 thia-3 tétrahydro-1,2,3,4 naphtylidène-2)-4 butène-2 oate d'éthyle fondant à 152°C.

Le diméthylamino-2 (oxo-1 thia-3 tétrahydro-1,2,3,4 naphtylidène-2)-4 butène-2 oate d'éthyle peut être préparé de la manière suivante :

On opère comme à l'exemple 3 pour la préparation du diméthylamino-2 (oxo-1 tétrahydro-1,2,3,4 naphtylidène-2)-4 butène-2 oate d'éthyle, à partir de 15,2 g de tétrafluoroborate de N-(diméthylamino-3 éthoxycarbonyl-3 propénylidène) N-méthyl méthanaminium, de 35 cm3 d'une solution éthanolique 2M d'éthylate de sodium et de 8,7 g d'oxo-1 thia-3 tétrahydro-1,2,3,4 naphtalène dans 160 cm3 d'éthanol. On obtient 13 g de diméthylamino-2 (oxo-1 thia-3 tétrahydro-1,2,3,4 naphtylidène-2)-4 butène-2 oate d'éthyle utilisé à l'état brut dans les synthèses ultérieures.

L'oxo-1 thia-3 tétrahydro-1,2,3,4 naphtalène peut être préparé selon le procédé suivant :

975 cm3 de nitrobenzène et 292,5 g d'acide polyphosphorique sont chauffés à une température voisine de 70°C. 97,5 g d'acide benzylthioacétique sont ajoutés au milieu réactionnel. On laisse agiter pendant 26 heures à environ 70°C puis refroidit à environ 20°C. 4000 cm3 d'une solution aqueuse saturée de bicarbonate de sodium et 4000 cm3 de dichlorométhane sont ajoutés à la solution. La phase organique est décantée et la phase aqueuse est extraite par 2 fois 200 cm3 de dichlorométhane. Les phases organiques sont réunies, séchées sur sulfate de magnésium anhydre, filtrées et évaporées à 40°C sous pression réduite (20 mm de mercure ; 2,7 kPa). Le résidu est dissous dans 80 cm3 d'un mélange de cyclohexane et d'acétate d'éthyle (85-15 en volumes) et la solution est versée sur 2 kg de silice contenus dans une colonne de 9 cm de diamètre. On élue avec un mélange de cyclohexane et d'acétate d'éthyle (85-15 en volumes) ; l'éluat correspondant est concentré à sec à 40°C sous pression réduite (20 mm de mercure ; 2,7 kPa). On obtient ainsi 10 g d'oxo-1 thia-3 tétrahydro-1,2,3,4 naphtalène utlisé à l'état brut dans les synthèses ultérieures.

L'acide benzylthioacétique peut être préparé de la manière suivante :

500 cm3 d'une solution aqueuse de soude 1N refroidie à une température d'environ 5°C sont ajoutés en 1 heure à 70 g d'acide bromoacétique. Le mélange obtenu est ajouté à une solution de 50 g de phénylméthanethiol, de 500 cm3 d'une solution aqueuse de soude 1N dans 500 cm3 de tétrahydrofuranne puis chauffé à environ 100°C pendant 90 minutes. Le mélange est refroidi à une température voisine de 5°C. On ajoute 100 cm3 d'une solution aqueuse d'acide chlorhydrique 12N puis 3000 cm3 de dichlorométhane en 30 minutes. La phase organique est décantée, la phase aqueuse est extraite par 2 fois 200 cm3 de dichlorométhane. Les phases organiques sont réunies, séchées sur du sulfate de magnésium anhydre, filtrées et évaporées à sec à 40°C sous pression réduite (20 mm de mercure ; 2,7 kPa). Le résidu est recristallisé dans 50 cm3 de cyclohexane bouillant. On obtient 40,5 g d'acide benzylthioacétique fondant à 58-60°C.

EXEMPLE 10

On opère comme à l'exemple 2, à partir de 6 g de diméthylamino-2 phénylthio-5 hexadiène-2,4 dioate de (1) éthyle et de (6) méthyle et de 30 cm3 d'une solution aqueuse d'acide chlorhydrique 12N. Le mélange est chauffé 1 minute à ébullition puis est refroidi à une température d'environ 20°C. Après purification par recristallisation dans 50 cm3 de cyclohexane bouillant, on obtient 4,3 d'hydroxy-2 phénylthio-5 hexadiène-2,4 dioate de (1) éthyle et de (6) méthyle fondant à 85°C.

Le diméthylamino-2 phénylthio-5 hexadiène-2,4 dioate de (1) éthyle et de (6) méthyle peut être préparé de la manière suivante :

On opère comme à l'exemple 2 pour la préparation du diméthylamino-2 phénylthio-5 hexadiène-2,4 dioate de diéthyle, à partir de 8,6 g de tétrafluoroborate de N-(diméthylamino-3 éthoxycarbonyl-3 propénylidène) N-méthyl méthanaminium, de 18,7 cm3 d'une solution 1,6 M de butyllithium dans l'hexane et de 5,5 g de phénylthioacétate de méthyle dans 50 cm3 de tétrahydrofuranne à une température voisine de 0°C. Après purification par chromatographie sur colonne de silice avec du dichlorométhane comme éluant et recristallisation dans 90 cm3 de cyclohexane bouillant, on obtient 6,8 g de diméthylamino-2 phénylthio-5 hexadiène-2,4 dioate de (1) éthyle et de (6) méthyle fondant à 75°C.

Le phénylthioacétate de méthyle peut être préparé de la façon suivante :

16 g d'acide phénylthioacétique, 0,26 cm3 d'une solution aqueuse d'acide sulfurique 36N dans 80 cm3 de méthanol sont portés à ébullition pendant 24 heures. Après refroidissement à environ 20°C, on ajoute au milieu réactionnel 1 g de carbonate de potassium. La phase organique est séchée sur sulfate de magnésium anhydre, filtrée et concentrée à sec à 50°C sous pression réduite (20 mm de mercure ; 2,7 kPa). L'huile est dissoute dans 20 cm3 d'un mélange de cyclohexane et d'acétate d'éthyle (50-50 en volumes) et la solution est versée sur 700 g de silice contenus dans une colonne de 7 cm de diamètre. On élue avec un mélange de cyclohexane et d'acétate d'éthyle (50-50 en volumes) ; l'éluat correspondant est concentré à sec à 40°C sous pression réduite (20 mm de mercure ; 2,7 kPa). On obtient ainsi 11 g de phénylthioacétate de méthyle utilisé à l'état brut dans les synthèses ultérieures.

EXEMPLE 11

On opère comme à l'exemple 4, à partir de 7,8 g de (chloro-4 phényl)-6 diméthylamino-2 méthylthio-5 oxo-6 hexadiène-2,4 oate d'éthyle et de 33 cm3 d'une solution aqueuse d'acide chlorhydrique 1N dans 300 cm3 d'éthanol. Le mélange est chauffé 5 minutes à ébullition puis refroidi à une température voisine de 20°C. Après purification par recristallisation dans 70 cm3 de cyclohexane bouillant, on obtient 5,6 g de (chloro-4 phényl)-6 hydroxy-2 méthylthio-5 oxo-6 hexadiène-2,4 oate d'éthyle fondant à 88°C.

Le (chloro-4 phényl)-6 diméthylamino-2 méthylthio-5 oxo-6 hexadiène-2,4 oate d'éthyle peut être préparé de la manière suivante :

On opère comme à l'exemple 4 pour la préparation du phényl-6 oxo-6 méthylthio-5 diméthylamino-2 hexadiène-2,4 oate d'éthyle, à partir de 11,3 g de tétrafluoroborate de N-(diméthylamino-3 éthoxycarbonyl-3 propénylidène) N-méthyl méthanaminium, de 19,7 cm3 d'une solution éthanolique 2M d'éthylate de sodium et de 7,9 g d'α-méthylthio chloro-4 acétophénone dans 86 cm3 d'éthanol. Après purification pa recristallisation dans 130 cm3 de cyclohexane bouillant, on obtient 7,8 g de (chloro-4 phényl)-6 diméthylamino-2 méthylthio-5 oxo-6 hexadiène-2,4 oate d'éthyle fondant à 93°C.

L'α-méthylthio chloro-4 acétophénone peut être préparée de la manière suivante :

A une solution agitée de 11,7 g d'α-bromo chloro-4 acétophénone dans 100 cm3 de méthanol, on ajoute, en 15 minutes, 3,5 g de méthanethiolate de sodium à une température voisine de 20°C et on laisse agiter pendant 2 heures à la même température. Le mélange réactionnel est évaporé à sec à 50°C sous pression réduite (20 mm de mercure ; 2,7 kPa). Le résidu obtenu est dissous dans 50 cm3 de dichlorométhane, la phase organique séchée sur du sulfate de magnésium anhydre, filtrée et concentrée à sec à 40°C sous pression réduite (20 mm de mercure ; 2,7 kPa). On obtient ainsi 9,9 g d'α-méthylthio chloro-4 acétophénone utilisée à l'état brut dans les synthèses ultérieures.

EXEMPLE 12

On opère comme à l'exemple 4 à partir de 4,1 g de diméthylamino-2 (méthyl-4 phényl)-6 méthylthio-5 oxo-6 hexadiène-2,4 oate d'éthyle et de 18,5 cm3 d'une solution aqueuse d'acide chlorhydrique 1N dans 18,5 cm3 d'éthanol. Le mélange est chauffé 5 minutes à ébullition puis refroidi à une température voisine de 20°C. Après purification par recristallisation dans 35 cm3 de cyclohexane bouillant, on obtient 2,9 g d'hydroxy-2 (méthyl-4 phényl)-6 méthylthio-5 oxo-6 hexadiène-2,4 oate d'éthyle fondant à 78°C.

Le diméthylamino-2 (méthyl-4 phényl)-6 méthylthio-5 oxo-6 hexadiène-2,4 oate d'éthyle peut être préparé de la manière suivante :

On opère comme à l'exemple 4 pour la préparation du phényl-6 oxo-6 méthylthio-5 diméthylamino-2 hexadiène-2,4 oate d'éthyle à partir de 23,4 g de tétrafluoroborate de N-(diméthylamino-3 éthoxycarbonyl-3 propénylidène) N-méthyl méthanaminium, de 40,8 cm3 d'une solution éthanolique 2M d'éthylate de sodium, et de 14,7 g d'α-méthylthio méthyl-4 acétophénone dans 163 cm3 d'éthanol. Après purification sur colonne de silice avec un mélange de cyclohexane et d'acétate d'éthyle (50-50 en volumes) comme éluant et recristallisation dans 65 cm3 de cyclohexane bouillant, on obtient 5,9 g de diméthylamino-2 (méthyl-4 phényl)-6 méthylthio-5 oxo-6 hexadiène-2,4 oate d'éthyle fondant à 98°C.

L'α-méthylthio méthyl-4 acétophénone peut être préparée de la manière suivante :

On opère comme à l'exemple 4 pour la préparation de l'α-méthylthio acétophénone ; à une solution agitée de 20,8 g d'α-bromo méthyl-4 acétophénone dans 195 cm3 de méthanol, on ajoute, en 30 minutes, 6,8 g de méthanethiolate de sodium, à une température voisine de 20°C et on laisse agiter pendant 2 heures à la même température. On obtient ainsi 16,5 g d'α-méthylthio méthyl-4 acétophénone utilisée à l'état brut dans les synthèses ultérieures.

EXEMPLE 13

On opère comme à l'exemple 4 à partir de 6,1 g de diméthylamino-2 (méthoxy-4 phényl)-6 méthylthio-5 oxo-6 hexadiène-2,4 oate d'éthyle et de 26,2 cm3 d'une solution aqueuse d'acide chlorhydrique 1N dans 26,2 cm3 d'éthanol. Le mélange est chauffé 5 minutes à ébullition puis refroidi à une température voisine de 20°C. Après purification par recristallisation dans 35 cm3 d'oxyde d'isopropyle bouillant, on obtient 2,7 g d'hydroxy-2 (méthoxy-4 phényl)-6 méthylthio-5 oxo-6 hexadiène-2,4 oate d'éthyle fondant à 88°C.

Le diméthylamino-2 (méthoxy-4 phényl)-6 méthylthio-5 oxo-6 hexadiène-2,4 oate d'éthyle peut être préparé de la manière suivante :

On opère comme à l'exemple 4 pour la préparation du phényl-6 oxo-6 méthylthio-5 diméthylamino-2 hexadiène-2,4 oate d'éthyle à partir de 13,9 g de tétrafluoroborate de N-(diméthylamino-3 éthoxycarbonyl-3 propénylidène) N-méthyl méthanaminium, de 24,2 cm3 d'une solution éthanolique 2M d'éthylate de sodium et de 9,5 g d'α-méthylthio méthoxy-4 acétophénone dans 97 cm3 d'éthanol. Après purification sur colonne de silice avec un mélange de cyclohexane et d'acétate d'éthyle (50-50 en volumes) comme éluant, on obtient 6,1 g de diméthylamino-2 (méthoxy-4 phényl)-6 méthylthio-5 oxo-6 hexadiène-2,4 oate d'éthyle utilisé à l'état brut dans les synthèses ultérieures.

L'α-méthylthio méthoxy-4 acétophénone peut être préparée de la façon suivante :

On opère comme à l'exemple 4 pour la préparation de l'α-méthylthio acétophénone à partir d'une solution agitée de 11,5 g d'α-bromo méthoxy-4 acétophénone dans 100 cm3 de méthanol, on ajoute en 15 minutes 3,5 g de méthanethiolate de sodium à une température voisine de 20°C et on laisse agiter pendant 12 heures à cette température. On obtient ainsi 9,5 g d'α-méthylthio méthoxy-4 acétophénone utilisé à l'état brut dans les

synthèses ultérieures.

EXEMPLE 14

On opère comme à l'exemple 4 à partir de 23,5 g de diméthylamino-2 méthylthio-5 oxo-6 (pipéridino-4 phényl)-6 hexadiène-2,4 oate d'éthyle et de 139 cm3 d'acide chlorhydrique 1N dans 139 cm3 d'éthanol. Le mélange est agité 16 heures à une température voisine de 20° C. Après purification par chromatographie sur colonne de silice avec du dichlorométhane comme éluant, puis par une seconde chromatographie avec un mélange de cyclohexane et d'acétate d'éthyle (50-50 en volumes) comme éluant ; l'éluat correspondant est concentré à sec à 40° C sous pression réduite (20 mm de mercure ; 2,7 kPa). Le résidu est recristallisé dans 100 cm3 d'oxyde d'isopropyle bouillant. On obtient ainsi 3 g d'hydroxy-2 méthylthio-5 oxo-6 (pipéridino-4 phényl)-6 hexadiène-2,4 oate d'éthyle fondant à 65° C.

Le diméthylamino-2 méthylthio-5 oxo-6 (pipéridino-4 phényl)-6 hexadiène-2,4 oate d'éthyle peut être obtenu de la manière suivante :

On opère comme à l'exemple 4 pour la préparation du phényl-6 oxo-6 méthylthio-5 diméthylamino-2 hexadiène-2,4 oate d'éthyle, à partir de 34,4 g de tétrafluoroborate de N-(diméthylamino-3 éthoxycarbonyl-3 propénylidène) N-méthyl méthanaminium, de 60 cm3 d'une solution éthanolique 2M d'éthylate de sodium et de 30 g d'α-méthylthio pipéridino-4 acétophénone dans 300 cm3 d'éthanol. Après purification par chromatographie sur colonne de silice avec un mélange de cyclohexane et d'acétate d'éthyle (50-50 en volumes) comme éluant et recristallisation dans 150 cm3 d'isopropanol bouillant, on obtient 27 g de diméthylamino-2 méthylthio-5 oxo-6 (pipéridino-4 phényl)-6 hexadiène-2,4 oate d'éthyle fondant à 113° C.

L'α-méthylthio pipéridino-4 acétophénone peut être préparée de la manière suivante :

On opère comme à l'exemple 4 pour la préparation de l'α-méthylthio acétophénone. A une solution agitée de 67 g d'α-chloro pipéridino-4 acétophénone dans 670 cm3 de méthanol, on ajoute, en 90 minutes, 20,1 g de méthanethiolate de sodium à une température voisine de 20° C et on continue l'agitation 3 heures à la même température. Après purification par recristallisation dans 1400 cm3 d'un mélange d'hexane et de cyclohexane (90-10 en volumes) bouillant, on obtient 55 g d'α-méthylthio pipéridino-4 acétophénone fondant à 66° C.

L'α-chloro pipéridino-4 acétophénone peut être préparée de la façon suivante :

A une solution agitée de 124 g d'α,α'-dibromo pipéridino-4 acétophénone dans 990 cm3 d'une solution aqueuse d'acide chlorhydrique 12N, on ajoute 620 cm3 de chlorure de titane III en solution aqueuse à 15 %. La solution est chauffée à ébullition pendant 2 heures 30 minutes puis refroidie à une température voisine de 20° C. Le mélange réactionnel est alcalinisé à pH 12 par 900 cm3 d'une solution aqueuse de soude 10N puis repris par 750 cm3 de dichlorométhane et filtré. La phase organique est décantée, lavée par 2 fois 200 cm3 d'eau, séchée sur sulfate de sodium, filtrée et évaporée à sec à 40° C sous pression réduite (20 mm de mercure ; 2,7 kPa). Le résidu est recristallisé dans 400 cm3 d'isopropanol bouillant. On obtient 67 g d'α-chloro pipéridino-4 acétophénone fondant à 125° C.

L'α,α'-dibromo pipéridino-4 acétophénone peut être préparée de la façon suivante :

A une solution agitée de 99.5 g de pipéridino-4 acétophénone dans 200 cm3 d'une solution aqueuse d'acide bromhydrique 8,6N, on ajoute, en 40 minutes, à une température voisine de 20° C, un mélange de 50 cm3 de brome et de 75 cm3 d'acide bromhydrique en conservant la température à environ 20° C. L'agitation est maintenue 2 heures à la même température. Le mélange réactionnel est filtré, le gâteau lavé par 300 cm3 d'eau. Le solide est broyé et repris par 800 cm3 de dichlorométhane. La solution obtenue est alcalinisée jusqu'à pH 11 par 200 cm3 de soude 10N et reprise par 600 cm3 d'eau. La phase organique est décantée, séchée sur sulfate de magnésium anhydre, filtrée et évaporée à sec à 40° C sous pression réduite (20 mm de mercure ; 2,7 kPa). Le résidu est recristallisé dans 2160 cm3 d'éthanol bouillant. On obtient 124 g d'α,α'-dibromo pipéridino-4 acétophénone fondant à 134° C.

EXEMPLE 15

On opère comme à l'exemple 5 à partir de 12,5 g de cyano-5 diméthylamino-2 phénylthio-5 pentadiène-2,4 oate d'éthyle et de 51,7 cm3 d'une solution d'éthanol chlorhydrique 4N. Le mélange est chauffé 3 minutes à ébullition puis refroidi à une température voisine de 20° C. Après purification par recristallisation dans 55 cm3 d'oxyde d'isopropyle bouillant, on obtient 3,2 g de cyano-5 hydroxy-2 phénylthio-5 pentadiène-2,4 oate d'éthyle fondant à 112° C.

Le cyano-5 diméthylamino-2 phénylthio-5 pentadiène-2,4 oate d'éthyle peut être préparé de la manière suivante :

On opère comme à l'exemple 2 à partir de 97 g de tétrafluoroborate de N-(diméthylamino-3 éthoxycarbonyl-3 propénylidène) N-méthyl méthanaminium, de 170 cm3 d'une solution éthanolique 2M d'éthylate de sodium et de 50,5 g de phénylthioacétonitrile dans 700 cm3 d'éthanol. Après purification par recristallisation dans 250 cm3 d'isopropanol bouillant, on obtient 21,4 g de diméthylamino-2 pénylthio-5 pentadiène-2,4 oate d'éthyle fondant à 88° C.

Le phénylthioacétonitrile peut être préparé de la manière suivante :

A une solution agitée de 20 g de thiophénol et 90 cm3 d'une solution éthanolique 2M d'éthylate de sodium, on ajoute, à une température voisine de 5° C, pendant 45 minutes, 11,4 cm3 de chloroacétonitrile. L'agitation est maintenue 5 heures à une température voisine de 20° C. Le mélange réactionnel est filtré, le filtrat est évaporé à sec à 50° C sous vide (20 mm de mercure ; 2,7 kPa). Le résidu est dissous dans 30 cm3 d'un mélange de cyclohexane et d'acétate d'éthyle (70-30 en volumes) et la solution est versée sur 1000 g de silice

contenus dans une colonne de 6,5 cm de diamètre. On élue avec un mélange de cyclohexane et d'acétate d'éthyle (70-30 en volumes) comme éluant. On obtient 14,5 g de phénylthioacétonitrile utilisé à l'état brut dans les phases ultérieures.

EXEMPLE 16

On opère comme à l'exemple 2 à partir de 10 g de (chloro-4 phénylthio)-5 diméthylamino-2 hexadiène-2,4 dioate de diéthyle, de 39 cm3 d'une solution aqueuse d'acide chlorhydrique 1N dans 39 cm3 d'éthanol. Le mélange est chauffé 2 minutes à ébullition puis refroidi à une température voisine de 20°C. Après purification par recristallisation dans 135 cm3 de cyclohexane bouillant, on obtient 6,8 g de (chloro-4 phénylthio)-5 hydroxy-2 hexadiène-2,4 dioate de diéthyle fondant à 84°C.

Le (chloro-4 phénylthio)-5 diméthylamino-2 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 14,3 g de tétrafluoroborate de N-(diméthylamino-3 éthoxycarbonyl-3 propénylidène) N-méthyl méthanaminium, de 25 cm3 d'une solution éthanolique 2M d'éthylate de sodium et de 11,5 g de (chloro-4 phénylthio) acétate d'éthyle dans 100 cm3 d'éthanol. On obtient ainsi 19 g de (chloro-4 phénylthio)-5 diméthylamino-2 hexadiène-2,4 dioate de diéthyle utilisé à l'état brut dans les phases ultérieures.

Le (chloro-4 phénylthio) acétate d'éthyle peut être préparé de la façon suivante :

On opère comme à l'exemple 2 pour la préparation du phénylthioacétate d'éthyle, à partir de 16,7 g de bromoacétate d'éthyle, de 50 cm3 d'une solution éthanolique 2M d'éthylate de sodium et de 14,5 g de chloro-4 thiophénol. On obtient ainsi 20,7 g de (chloro-4 phénylthio) acétate d'éthyle utilisé à l'état brut dans les phases ultérieures.

EXEMPLE 17

On opère comme à l'exemple 2 à partir de 10 g de diméthylamino-2 (méthoxy-4 phénylthio)-5 hexadiène-2,4 dioate de diéthyle et de 39,5 cm3 d'une solution aqueuse d'acide chlorhydrique 1N dans 39,5 cm3 d'éthanol. Le mélange est chauffé 3 minutes à ébullition puis refroidi à une température voisine de 20°C. Après purification par chromatographie sur colonne de silice avec un mélange de cyclohexane et d'acétate d'éthyle (50-50 en volumes) comme éluant et recristallisation dans 105 cm3 de cyclohexane bouillant, on obtient 6 g d'hydroxy-2 (méthoxy-4 phénylthio)-5 hexadiène-2,4 dioate de diéthyle fondant à 88°C.

Le diméthylamino-2 (méthoxy-4 phénylthio)-5 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 17,2 g de tétrafluoroborate de N-(diméthylamino-3 éthoxycarbonyl-3 propénylidène) N-méthyl méthanaminium, de 30 cm3 d'une solution éthanolique 2M d'éthylate de sodium et de 13,6 g de (méthoxy-4 phénylthio) acétate d'éthyle dans 136 cm3 d'éthanol. Après purification par recristallisation dans 110 cm3 d'oxyde d'isopropyle bouillant, on obtient 8,4 g de diméthylamino-2 (méthoxy-4 phénylthio)-5 hexadiène-2,4 dioate de diéthyle fondant à 78°C.

Le (méthoxy-4 phénylthio) acétate d'éthyle peut être préparé de la façon suivante :

On opère comme à l'exemple 2 pour la préparation du phénylthioacétate d'éthyle, à partir de 11,7 g de bromoacétate d'éthyle, de 35 cm3 d'une solution éthanolique 2M d'éthylate de sodium et de 10 g de méthoxy-4 thiophénol. On obtient ainsi 13,6 g de (méthoxy-4 phénylthio) acétate d'éthyle utilisé à l'état brut dans les synthèses ultérieures.

EXEMPLE 18

On opère comme à l'exemple 2, à partir de 10 g de benzylthio-5 diméthylamino-2 hexadiène-2,4 dioate de diéthyle et de 41,3 cm3 d'une solution aqueuse d'acide chlorhydrique 1N dans 41,3 cm3 d'éthanol. Le mélange est chauffé 2 minutes à ébullition puis refroidi à une température voisine de 20°C. Après purification par chromatographie sur colonne de silice avec un mélange de cyclohexane et d'acétate d'éthyle (30-70 en volumes) comme éluant, on obtient 6,3 g de benzylthio-5 hydroxy-2 hexadiène-2,4 dioate de diéthyle (point de fusion < 40°C) [Rf=0,6 ; chromatographie sur couche mince de gel de silice ; éluant : cyclohexane-acétate d'éthyle (30-70 en volumes)].

Le benzylthio-5 diméthylamino-2 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 15,7 g de tétrafluoroborate de N-(diméthylamino-3 éthoxycarbonyl-3 propénylidène) N-méthyl méthanaminium, de 27,5 cm3 d'une solution éthanolique 2M d'éthylate de sodium et de 11,6 g de benzylthioacétate d'éthyle dans 110 cm3 d'éthanol. On obtient ainsi 19,2 g de benzylthio-5 diméthylamino-2 hexadiène-2,4 dioate de diéthyle utilisé à l'état brut dans les phases ultérieures.

Le benzylthioacétate d'éthyle peut être préparé de la façon suivante :

On opère comme à l'exemple 2 pour la préparation du phénylthioacétate d'éthyle, à partir de 16,7 g de bromoacétate d'éthyle, de 50 cm3 d'une solution éthanolique 2M d'éthylate de sodium et de 11,7 cm3 de benzylmercaptan. On obtient ainsi 19,4 g de benzylthioacétate d'éthyle utilisé à l'état brut dans les phases ultérieures.

EXEMPLE 19

On opère comme à l'exemple 2 à partir de 5,1 g d'allylthio-5 diméthylamino-2 hexadiène-2,4 dioate de diéthyle et de 24,4 cm3 d'une solution aqueuse d'acide chlorhydrique 1N dans 24,4 cm3 d'éthanol. Le mélange est chauffé 3 minutes à ébullition puis refroidi à une température voisine de 20°C. Après purification par chromatographie sur colonne de silice avec un mélange de cyclohexane et d'acétate d'éthyle (75-25 en volumes) comme éluant, on obtient 2,3 g d'allylthio-5 hydroxy-2 hexadiène-2,4 dioate de diéthyle sous forme d'une huile jaune [Rf = 0,2 ; chromatographie sur couche mince de gel de silice ; éluant : cyclohexane-acétate d'éthyle (75-25 en volumes)].

L'allylthio-5 diméthylamino-2 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 5,5 g de tétrafluoroborate de N-(diméthylamino-3 éthoxycarbonyl-3 propénylidène) N-méthyl méthanaminium, de 9,7 cm3 d'une solution éthanolique 2M d'éthylate de sodium et de 3,1 g d'allylthioacétate d'éthyle dans 31 cm3 d'éthanol. On obtient ainsi 5,1 g d'allylthio-5 diméthylamino-2 hexadiène-2,4 dioate de diéthyle utilisé à l'état brut dans les phases ultérieures.

L'allylthioacétate d'éthyle peut être préparé de la manière suivante :

On opère comme à l'exemple 2 pour la préparation du phénylthioacétate d'éthyle à partir de 16,7 g de bromoacétate d'éthyle, de 50 cm3 d'une solution éthanolique 2M d'éthylate de sodium et de 7,7 cm3 d'allylmercaptan. Après purification par chromatographie sur colonne de silice avec du cyclohexane comme éluant, on obtient 3,1 g d'allylthioacétate d'éthyle utilisé à l'état brut dans les phases ultérieures.

EXEMPLE 20

On opère comme à l'exemple 2 à partir de 9,3 g de diméthylamino-2 éthoxycarbonyl-5 tridécadiène-2,4 oate d'éthyle et de 40 cm3 d'une solution aqueuse d'acide chlorhydrique 1N dans 40 cm3 d'éthanol. Le mélange est chauffé 5 minutes à ébullition puis refroidi à une température voisine de 20°C. Après purification par recristallisation dans 100 cm3 d'éther de pétrole 40-65°, on obtient 3,9 g d'éthoxycarbonyl-5 hydroxy-2 tridécadiène-2,4 oate d'éthyle fondant à 58°C.

Le diméthylamino-2 éthoxycarbonyl-5 tridécadiène-2,4 oate d'éthyle peut être préparé de la manière suivante :

A un mélange agité de 5,6 cm3 de diisopropylamine, de 37,5 cm3 d'une solution 1,6M de n-butyllithium dans l'hexane et de 30 cm3 de tétrahydrofuranne, on ajoute, à une température voisine de -78°C, en 10 minutes, 10 g de n-décanoate d'éthyle. L'agitation est maintenue 30 minutes à la même température puis on ajoute 14,3 g de tétrafluoroborate de N-(diméthylamino-3 éthoxycarbonyl-3 propénylidène) N-méthyl méthanaminium et maintient l'agitation 3 heures à une température voisine de -78°C. Après réchauffement progressif à environ 20°C, on ajoute au milieu réactionnel 4 g de chlorure d'ammonium dissous dans 50 cm3 d'eau. On ajoute 200 cm3 de dichlorométhane et 100 cm3 d'eau et décante la phase organique. La phase aqueuse est lavée avec 2 fois 50 cm3 de dichlorométhane. Les phases organiques sont réunies, séchées sur sulfate de magnésium anhydre, filtrées et évaporées à 40°C sous pression réduite (20 mm de mercure ; 2,7 kPa). Le résidu est dissous dans 30 cm3 d'un mélange de cyclohexane et d'acétate d'éthyle (60-40 en volumes) et la solution obtenue est versée sur 600 g de silice contenus dans une colonne de 7 cm de diamètre. On élue avec un mélange de cyclohexane et d'acétate d'éthyle (60-40 en volumes) et l'éluat est concentré à sec à 40°C sous pression réduite (20 mm de mercure ; 2,7 kPa). On obtient ainsi 9,8 g de diméthylamino-2 éthoxycarbonyl-5 tridécadiène-2,4 oate d'éthyle utilisé à l'état brut dans les synthèses ultérieures.

Le n-décanoate d'éthyle peut être préparé de la manière suivante :

17,2 g d'acide n-décanoïque, 3 cm3 d'une solution aqueuse d'acide sulfurique 36N et 300 cm 3 d'éthanol sont chauffés 4 heures à ébullition. Après refroidissement à une température voisine de 20°C, la solution est concentrée à sec à 50°C sous vide (20 mm de mercure ; 2,7 kPa) puis le résidu est repris par 200 cm3 de dichlorométhane. La phase organique est lavée avec 2 fois 100 cm3 d'une solution aqueuse saturée de bicarbonate de sodium, décantée, séchée sur sulfate de magnésium anhydre, filtrée et concentrée à sec à 40°C sous pression réduite (20 mm de mercure ; 2,7 kPa). On obtient ainsi 18 g de n-décanoate d'éthyle utilisé à l'état brut dans les synthèses ultérieures.

EXEMPLE 21

On opère comme à l'exemple 2, à partir de 5 g de diméthylamino-5 phénylthio-2 pentadiène-2,4 oate d'éthyle et de 108 cm3 d'une solution aqueuse d'acide chlorhydrique 1N dans 90 cm3 d'éthanol. Le mélange est chauffé 2 minutes à ébullition puis refroidi à une température voisine de 20°C. Après purification par recristallisation dans 15 cm3 d'un mélange de cyclohexane et d'acétate d'éthyle bouillant (50-50 en volumes), on obtient 1,5 g d'hydroxy-5 phénylthio-2 pentadiène-2,4 oate d'éthyle fondant à 120°C.

Le diméthylamino-5 phénylthio-2 pentadiène-2,4 oate d'éthyle peut être préparé de la manière suivante :

A une solution agitée de 25,1 g de phénylthioacétate d'éthyle dans 280 cm3 d'éthanol maintenue sous atmosphère d'azote, on ajoute, à une température voisine de 20°C, pendant 15 minutes, 64 cm3 d'une solution éthanolique 2M d'éthylate de sodium. L'agitation est maintenue 30 minutes à la même température puis 20,8 g de chlorure de N-(diméthylamino-3 propénylidène) N-méthyl méthanaminium sont ajoutés. Le milieu réactionnel est agité 15 heures à une température voisine de 20°C. Le mélange est concentré à sec à 40°C sous pression réduite (320 mm de mercure ; 2,7 kPa) puis repris par 200 cm3 d'eau. Le précipité formé est filtré, lavé par 50 cm3 d'eau et recristallisé dans 125 cm3 d'éthanol bouillant. On obtient 18,1 g de

diméthylamino-5 phénylthio-2 pentadiène-2,4 oate d'éthyle fondant à 128° C.

Le chlorure de N-(diméthylamino-3 propénylidène) N-méthyl méthanaminium peut être préparé selon la méthode décrite par V. NAIR et C.S. COOPER, J. Org. Chem., 46, 4759 (1981).

EXEMPLE 22

A une solution agitée de 5 g d'hydroxy-2 phénylthio-5 hexadiène-2,4 dioate de diéthyle et 2,4 cm3 de triéthylamine dans 45 cm3 de tétrahydrofuranne, traversée par un courant d'azote, on ajoute, à une température d'environ 20° C et pendant 5 minutes, 1,2 cm3 de chlorure d'acétyle. L'agitation est maintenue 2 heures à la même température. Le mélange est repris par 100 cm3 d'eau et 100 cm3 de dichlorométhane. La phase organique est décantée et la phase aqueuse est extraite par 2 fois 50 cm3 de dichlorométhane. Les phases organiques sont rassemblées, séchées sur du sulfate de magnésium anhydre, filtrées et concentrées à sec à 40° C sous pression réduite (20 mm de mercure ; 2,7 kPa). L'huile obtenue est mise en solution dans 10 cm3 d'un mélange de cyclohexane et d'acétate d'éthyle (70-30 en volumes) et versée sur 300 g de silice contenus dans une colonne de 4 cm de diamètre. On élue avec un mélange de cyclohexane et d'acétate d'éthyle (70-30 en volumes) et l'éluat corespondant est concentré à sec à 40° C sous pression réduite (20 mm de mercure ; 2,7 kPa). On obtient 4,5 g d'acétoxy-2 phénylthio-5 hexadiène-2,4 dioate de diéthyle sous forme d'une huile orangée [Rf=0,50 ; chromatographie sur couche mince de gel de silice ; éluant : cyclohexane-acétate d'éthyle (70-30 en volumes)].

EXEMPLE 23

On opère comme à l'exemple 2, à partir de 4,5 g de diméthylamino-2 phénylthio-5 hexadiène-2,4 dioate de (1) éthyle et de (6) cyclohexyle et de 0,5 cm3 d'une solution aqueuse d'acide chlorhydrique 12N dans 50 cm3 de tétrahydrofuranne. La solution est agitée 15 heures à une température d'environ 20° C. Après purification par recristallisation dans 70 cm3 de cyclohexane bouillant, on obtient 2,3 g d'hydroxy-2 phénylthio-5 hexadiène-2,4 dioate de (1) éthyle et de (6) cyclohexyle fondant à 110° C.

Le diméthylamino-2 phénylthio-5 hexadiène-2,4 dioate de (1) éthyle et de (6) cyclohexyle peut être préparé de la manière suivante :

On opère comme à l'exemple 2 pour la préparation du diméthylamino-2 phénylthio-5 hexadiène-2,4 dioate de diéthyle, à partir de 13,7 g de tétrafluoroborate de N-(diméthylamino-3 éthoxycarbonyl-3 propénylidène) N-méthyl méthanaminium, de 33,1 cm3 d'une solution 1,6M dans l'hexane de n-butyllithium et de 12 g de phénylthioacétate de cyclohexyle dans 150 cm3 de tetrahydrofuranne. Après purification par chromatographie sur colonne de silice avec un mélange de cyclohexane et d'acétate d'éthyle (80-20 en volumes) comme éluant, on obtient 9,7 g de diméthylamino-2 phénylthio-5 hexadiène-2,4 dioate de (1) éthyle et de (6) cyclohexyle utilisé à l'ébat brut dans les phases ultérieures.

Le phénylthioacétate de cyclohexyle peut être préparé de la manière suivante :

On opère comme à l'exemple 20 pour la préparation du n-décanoate d'éthyle, à partir de 20 g d'acide phénylthioacétique et de 0,32 cm3 d'une solution aqueuse d'acide sulfurique 36N dans 100 cm3 de cyclohexanol. Après purification par chromatographie sur colonne de silice avec un mélange de cyclohexane et d'acétate d'éthyle (70-30 en volumes) comme éluant, on obtient 29 g de phénylthioacétate de cyclohexyle utilisé à l'état brut dans les phases ultérieures.

EXEMPLE 24

On opère comme à l'exemple 2 à partir de 3,5 g de diméthylamino-5 phényl-5 phénylthio-2 pentadiène-2,4 oate d'éthyle et de 17 cm3 d'une solution aqueuse d'acide chlorhydrique 1N dans 17 cm3 d'éthanol. La solution est chauffée 2 minutes à ébullition puis refroidie à une température voisine de 20° C. Après purification par chromatographie sur colonne de silice avec du dichlorométhane comme éluant, on obtient 1,7 g d'oxo-5 phényl-5 phénylthio-2 pentène-2 oate d'éthyle sous forme d'une huile jaune (Rf=0,40 ; chromatographie sur couche mince de gel de silice ; éluant : dichlorométhane).

Le diméthylamino-5 phényl-5 phénylthio-2 pentadiène-2,4 oate d'éthyle peut être préparé de la manière suivante :

On opère comme à l'exemple 2 pour la préparation du diméthylamino-2 phénylthio-5 hexadiène-2,4 dioate de diéthyle, à partir de 15,4 g de tétrafluoroborate de N-(diméthylamino-3 phényl-3 propénylidène) N-méthyl méthanaminium, de 26,5 cm3 d'une solution 2M d'éthylate de sodium et de 10,4 g de phénylthioacétate d'éthyle dans 150 cm3 d'ethanol. Après purification par chromatographie sur colonne de silice avec du dichlorométhane comme éluant, on obtient 9,7 g de diméthylamino-5 phényl-5 phénylthio-2 pentadiène-2,4 oate d'éthyle fondant à 99° C.

Le tétrafluoroborate de N-(diméthylamino-3 phényl-3 propénylidène) N-méthyl méthanaminium peut être préparé de la manière suivante :

On opère comme à l'exemple 2 pour la préparation du tétrafluoroborate N-(éthoxy-3 éthoxycarbonyl-3 propénylidène) N-méthyl méthanaminium et du tétrafluoroborate de N-(diméthylamino-3 éthoxycarbonyl-3 propénylidène) N-méthyl méthanaminium, à partir de 10,1 g de diméthylamino-3 phényl-1 propène-2 one-1, de 12,3 g de tétrafluoroborate de triéthyloxonium et de 5 cm3 de diméthylamine dans 50 cm3 de dichlorométhane. Après purification par recristallisation dans 100 cm3 d'isopropanol bouillant, on obtient 15,4 g de tétrafluoroborate de N-(diméthylamino-3 phényl-3 propénylidène) N-méthyl méthanaminium fondant à 136° C.

La diméthylamino-3 phényl-1 propène-2 one-1 peut être préparée selon la méthode décrite par H.

BRADERECK, F. EFFARBERGER et H. BOTSCH, Chem. Ber. 97, 3397 (1964).

EXEMPLE 25

On opère comme à l'exemple 2, à partir de 4,6 g de diméthylamino-2 phényl-5 hexadiène-2,4 dioate de diéthyle et de 28 cm3 d'une solution aqueuse d'acide chlorhydrique 1N dans 28 cm3 d'éthanol. La solution est agitée à une température voisine de 20° C pendant 15 heures. Après purification par recristallisation dans 35 cm3 de cyclohexane bouillant, on obtient 1,8 g d'hydroxy-2 phényl-5 hexadiène-2,4 dioate de diéthyle fondant à 107° C.

Le diméthylamino-2 phényl-5 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 10 g de tétrafluoroborate de N-(diméthylamino-3 éthoxycarbonyl-3 propénylidène) N-méthyl méthanaminium, de 21 cm3 d'une solution éthanolique 2M d'éthylate de sodium et de 5,6 cm3 de phénylacétate d'éthyle dans 100 cm3 d'éthanol. Après purification par chromatographie sur colonne de silice avec un mélange de cyclohexane et d'acétate d'éthyle (80-20 en volumes) comme éluant, on obtient 4,6 g de diméthylamino-2 phényl-5 hexadiène-2,4 dioate de diéthyle utilisé à l'état brut dans les phases ultérieures.

EXEMPLE 26

On opère comme à l'exemple 4, à partir de 4,9 g de diméthylamino-2 (isopropylènedioxy-3,4 phényl)-6 méthylthio-5 oxo-6 hexadiène-2,4 oate d'éthyle et de 20 cm3 d'une solution aqueuse d'acide chlorhydrique 1N dans 20 cm3 d'éthanol. Le mélange est chauffé 5 minutes à ébullition puis refroidi à une température voisine de 20° C. Après purification par recristallisation dans 70 cm3 d'oxyde d'isopropyle bouillant, on obtient 1,1 g d'hydroxy-2 (isopropylènedioxy-3,4 phényl)-6 méthylthio-5 oxo-6 hexadiène-2,4 oate d'éthyle fondant à 134° C.

Le diméthylamino-2 (isopropylènedioxy-3,4 phényl)-6 méthylthio-5 oxo-6 hexadiène-2,4 oate d'éthyle peut être préparé de la manière suivante :

On opère comme à l'exemple 4 pour la préparation du phényl-6 oxo-6 méthylthio-5 diméthylamino-2 hexadiène-2,4 oate d'éthyle, à partir de 7,2 g de tétrafluoroborate de N-(diméthylamino-3 éthoxycarbonyl-3 propénylène) N-méthyl méthanaminium, de 12,5 cm3 d'une solution éthanolique 2M d'éthylate de sodium et de 6 g de diméthyl-2,2 (méthylthio-2 acétyl)-5 benzodioxole-1,3 dans 50 cm3 d'éthanol. Après purification sur colonne de silice avec un mélange de cyclohexane et d'acétate d'éthyle (50-50 en volumes) comme éluant, on obtient 3,9 g de diméthylamino-2 (isopropylènedioxy-3,4 phényl)-6 méthylthio-5 oxo-6 hexadiène-2,4 oate d'éthyle utilisé à l'état brut dans les synthèses ultérieures.

Le diméthyl-2,2 (méthylthio-2 acétyl)-5 benzodioxole-1,3 peut être préparé de la manière suivante :

On opère comme à l'exemple 4 pour la préparation de l'α-méthylthioacétophénone, à partir d'une solution agitée de 14,9 g de (bromo-2 acétyl)-5 diméthyl-2,2 benzodioxole-1,3 dans 150 cm3 de méthanol. On ajoute, en 5 minutes, 3,8 g de méthanethiolate de sodium, à une température voisine de 20° C et on laisse agiter 15 heures à la même température. On obtient ainsi 11 g de diméthyl-2,2 (méthylthio-2 acétyl)-5 benzodioxole-1,3 utilisé à l'état brut dans les synthèses ultérieures.

Le (bromo-2 acétyl)-5 diméthyl-2,2 benzodioxole-1,3 peut être préparé de la manière suivante :

A une solution agitée de 5,8 g d'acétyl-5 diméthyl-2,2 benzodioxole-1,3 dans 100 cm3 de tétrachlorure de carbone, on ajoute en 5 minutes, 1,5 cm3 de brome à une température voisine de 20° C et on laisse agiter 15 heures à la même température. Le mélange réactionnel est repris pas 150 cm3 d'eau. La phase organique est décantée, séchée sur sulfate de magnésium anhydre, filtrée et évaporée à sec à 40° C sous pression réduite (20 mm de mercure ; 2,7 kPa). On obtient 7,1 g de (bromo-2 acétyl)-5 diméthyl-2,2 benzodioxole-1,3 utilisé à l'état brut dans les synthèses ultérieures.

L'acétyl-5 diméthyl-2,2 benzodioxole-1,3 peut être préparé selon la méthode décrite par G. BENOIT et B. MILLET, Bull. Soc. Chim. Fr. 1960, 638.

EXEMPLE 27

On opère comme à l'exemple 4, à partir de 1,9 g de (dihydroxy-3,4 phényl)-6 diméthylamino-2 méthylthio-5 oxo-6 hexadiène-2,4 oate d'éthyle et de 10,8 cm3 d'une solution aqueuse d'acide chlorhydrique 1N dans 10,8 cm3 d'éthanol. Le mélange est chauffé 5 minutes à ébullition puis refroidi à une température voisine de 20° C. Après purification par cristallisation dans 100 cm3 d'oxyde d'isopropyle, on obtient 0,6 g de (dihydroxy-3,4 phényl)-6 hydroxy-2 méthylthio-5 oxo-6 hexadiène-2,4 oate d'éthyle fondant à 156° C.

Le (dihydroxy-3,4 phényl)-6 diméthylamino-2 méthylthio-5 oxo-6 hexadiène-2,4 oate d'éthyle peut être préparé de la manière suivante :

On opère comme à l'exemple 4 pour la préparation du phényl-6 oxo-6 méthylthio-5 diméthylamino-2 hexadiène-2,4 oate d'éthyle, à partir de 11,4 g de tétrafluoroborate de N-(diméthylamino-3 éthoxycarbonyl-3 propénylidène) N-méthyl méthanaminium, de 60 cm3 d'une solution éthanolique 2M d'éthylate de sodium et de 7, g de (méthylthio-2 acétyl)-4 pyrocatéchol dans 60 cm3 d'éthanol. Après purification sur colonne de silice avec un mélange de cyclohexane et d'acétate d'éthyle (50-50 en volumes) comme éluant, on obtient 2,4 g de (dihydroxy-3,4 phényl)-6 diméthylamino-2 méthylthio-5 oxo-6 hexadiène-2,4 oate d'éthyle fondant à 144° C.

Le (méthylthio-2 acétyl)-4 pyrocatéchol peut être préparé de la manière suivante :

On opère comme à l'exemple 4 pour la préparation de l'α-méthylthioacétophénone, à partir d'une solution agitée de 18,6 g de (chloro-2 acétyl)-4 pyrocatéchol dans 200 cm3 de méthanol. On ajoute, en 15 minutes, 7 g de méthanethiolate de sodium, à une température voisine de 20° C et on laisse agiter 3 heures à la même

température. On obtient ainsi 14,5 g de (méthylthio-2 acétyl)-4 pyrocatéchol fondant à 107° C.

EXEMPLE 28

On opère comme à l'exemple 4, à partir de 8 g de diméthylamino-2 méthoxy-5 oxo-6 phényl-6 hexadiène-2,4 oate d'éthyle et de 40 cm3 d'une solution aqueuse d'acide chlorhydrique 1N dans 40 cm3 d'éthanol. Le mélange est chauffé 5 minutes à ébullition puis refroidi à une température voisine de 20° C. Après purification par chromatographie sur colonne de silice avec un mélange de cyclohexane et d'acétate d'éthyle (50-50 en volumes) comme éluant et recristallisation dans 70 cm3 d'oxyde d'isopropyle bouillant, on obtient 4 g d'hydroxy-2 méthoxy-5 oxo-6 phényl-6 hexadiène-2,4 oate d'éthyle fondant à 84° C.

Le diméthylamino-2 méthoxy-5 oxo-6 phényl-6 hexadiène-2,4 oate d'éthyle peut être préparé de la manière suivante :

On opère comme à l'exemple 4 pour la préparation du phényl-6 oxo-6 méthylthio-5 diméthylamino-2 hexadiène-2,4 oate d'éthyle, à partir de 11,4 g de tétrafluoroborate de N-diméthylamino-3 éthoxycarbonyl-3 propénylidène) N-méthyl méthanaminium, de 20 cm3 d'une solution éthanolique 2M d'éthylate de sodium et de 6 g d'α-méthoxyacétophénone dans 50 cm3 d'éthanol. Après purification par chromatographie sur colonne de silice avec un mélange de dichlorométhane et d'acétate d'éthyle (50-50 en volumes) comme éluant, on obtient 8,1 g de diméthylamino-2 méthoxy-5 oxo-6 phényl-6 hexadiène-2,4 oate d'éthyle utilisé à l'état brut dans les synthèses ultérieures.

L'α-méthoxyacétophénone est préparée selon R.B. MOFFETT et R.L. SHRINER, Org. Synth. 3, 562 (1955).

EXEMPLE 29

On opère comme à l'exemple 2, à partir de 5 g de diméthylamino-2 (méthyl-4 benzylthio)-5 hexadiène-2,4 dioate de diéthyle, de 20 cm3 d'une solution aqueuse d'acide chlorhydrique 12N et de 20 cm3 d'éthanol. Le mélange est chauffé 1 minute à ébullition puis est refroidi à une température d'environ 20° C. Après purification par recristallisation dans 84 cm3 d'éther de pétrole (40-60°) bouillant, on obtient 3,2 g d'hydroxy-2 (méthyl-4 benzylthio)-5 hexadiène-2,4 dioate de diéthyle fondant à 66° C.

Le diméthylamino-2 (méthyl-4 benzylthio)-5 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 5,7 g de tétrafluoroborate de N-(diméthylamino-3 éthoxycarbonyl-3 propénylidène) N-méthyl méthanaminium, de 10 cm3 d'une solution éthanolique 2M d'éthylate de sodium et de 4,5 g de (méthyl-4 benzylthio) acétate d'éthyle dans 50 cm3 d'éthanol. Après purification par chromatographie sur colonne de silice avec un mélange de cyclohexane et d'acétate d'éthyle (50-50 en volumes) comme éluant, on obtient 5 g de diméthylamino-2 (méthyl-4 benzylthio)-5 hexadiène-2,4 dioate de diéthyle, sous forme d'une huile jaune, utilisé à l'état brut dans les synthèses ultérieures.

Le (méthyl-4 benzylthio) acétate d'éthyle peut être préparé de la façon suivante :

A une solution agitée de 37,5 cm3 d'une solution éthanolique 2M d'éthylate de sodium et de 50 cm3 d'éthanol, traversée par un courant d'azote, on ajoute en 15 minutes, 8,5 cm3 de mercapto-2 acétate d'éthyle à une température voisine de 20° C. On laisse agiter 15 minutes à la même température puis on ajoute en 20 minutes 13,9 g d'α-bromo p-xylène à une température voisine de 20° C. L'agitation est maintenue 60 minutes à cette température puis le mélange réactionnel est évaporé à sec à 50° C sous pression réduite (20 mm de mercure ; 2,7 kPa). Le résidu obtenu est dissous dans 100 cm3 de dichlorométhane, la phase organique séchée sur du sulfate de magnésium anhydre, filtrée et concentrée à sec à 40° C sous pression réduite (20 mm de mercure ; 2,7 kPa). On obtient ainsi 15,6 g de (méthyl-4 benzylthio) acétate d'éthyle utilisé à l'état brut dans les synthèses ultérieures.

EXEMPLE 30

On opère comme à l'exemple 2, à partir de 6 g de diméthylamino-2 (phényl-4 benzylthio)-5 hexadiène-2,4 dioate de diéthyle, de 20,5 cm3 d'une solution aqueuse d'acide chlorhydrique 12N et de 40 cm3 d'éthanol. Le mélange est chauffé 1 minute à ébullition puis est refroidi à une température d'environ 20° C. Après purification par recristallisation dans 70 cm3 d'isopropanol bouillant, on obtient 4,25 g d'hydroxy-2 (phényl-4 benzylthio)-5 hexadiène-2,4 dioate de diéthyle fondant à 133° C.

Le diméthylamino-2 (phényl-4 benzylthio)-5 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 5,7 g de tétrafluoroborate de N-diméthylamino-3 éthoxycarbonyl-3 propénylidène) N-méthyl méthanaminium, de 10 cm3 d'une solution éthanolique 2M d'éthylate de sodium et de 5,7 g de (phényl-4 benzylthio) acétate d'éthyle dans 50 cm3 d'éthanol. Après purification par chromatographie sur colonne de silice avec un mélange de cyclohexane et d'acétate d'éthyle (70-30 en volumes) comme éluant, on obtient 6 g de diméthylamino-2 (phényl-4 benzylthio)-5 hexadiène-2,4 dioate de diéthyle, sous forme d'une huile jaune, utilisé à l'état brut dans les synthèses ultérieures.

Le (phényl-4 benzylthio) acétate d'éthyle peut être préparé de la façon suivante :

On opère comme à l'exemple 29 pour la préparation du (méthyl-4 benzylthio) acétate d'éthyle, à partir de 10,25 g de chlorométhyl-4 biphényle, de 25 cm3 d'une solution éthanolique 2M d'éthylate de sodium et de 6,1 g de mercapto-2 acétate d'éthyle dans 150 cm3 d'éthanol. On obtient ainsi 13,1 g de phényl-4 benzylthio acétate

d'éthyle utilisé à l'état brut dans les synthèses ultérieures.

EXEMPLE 31

On opère comme à l'exemple 2, à partir de 7 g de diméthylamino-2 (phénoxy-3 benzylthio)-5 hexadiène-2,4 dioate de diéthyle, de 23 cm3 d'une solution aqueuse d'acide chlorhydrique 12N et de 46 cm3 d'éthanol. Le mélange est chauffé 1 minute à ébullition puis est refroidi à une température d'environ 20° C. Après purification par chromatographie sur colonne de silice avec du dichlorométhane comme éluant, on obtient 4,5 g d'hydroxy-2 (phénoxy-3 benzylthio)-5 hexadiène-2,4 dioate de diéthyle, sous forme d'une huile jaune (Rf=0,2 ; chromatographie sur couche mince de gel de silice ; éluant : dichlorométhane).

Le diméthylamino-2 (phénoxy-3 benzylthio)-5 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 5,7 g de tétrafluoroborate de N-(diméthylamino-3 éthoxycarbonyl-3 propénylidène) N-méthyl méthanaminium, de 10 cm3 d'une solution éthanolique 2M d'éthylate de sodium et de 6,1 g de (phénoxy-3 benzylthio) acétate d'éthyle dans 50 cm3 d'éthanol. Après purification par chromatographie sur colonne de silice avec un mélange de cyclohexane et d'acétate d'éthyle (50-50 en volumes) comme éluant, on obtient 7 g de diméthylamino-2 (phénoxy-3 benzylthio)-5 hexadiène-2,4 dioate de diéthyle, sous forme d'une huile jaune, utilisé à l'état brut dans les synthèses ultérieures.

Le (phénoxy-3 benzylthio) acétate d'éthyle peut être préparé de la façon suivante :

On opère comme à l'exemple 29 pour la préparation du (méthyl-4 benzylthio) acétate d'éthyle, à partir de 21,5 g de chlorométhyl-1 phénoxy-4 benzène, de 50 cm3 d'une solution éthanolique 2M d'éthylate de sodium et de 12,2 g de mercapto-2 acétate d'éthyle dans 200 cm3 d'éthanol. On obtient ainsi 28,8 g de (phénoxy-3 benzylthio) acétate d'éthyle utilisé à l'état brut dans les synthèses ultérieures.

EXEMPLE 32

On opère comme à l'exemple 4, à partir de 6,9 g de diméthylamino-2 méthylthio-5 (naphtyl-2)-6 oxo-6 hexadiène-2,4 oate d'éthyle, de 29 cm3 d'une solution aqueuse d'acide chlorhydrique 12N et de 29 cm3 d'éthanol. Le mélange est chauffé 5 minute à ébullition puis est refroidi à une température d'environ 20° C. Après purification par recristallisation dans 300 cm3 d'oxyde de diisopropyle bouillant, on obtient 2,7 g d'hydroxy-2 méthylthio-5 (naphtyl-2)-6 oxo-6 hexadiène-2,4 oate d'éthyle fondant à 117° C.

Le diméthylamino-2 méthylthio-5 (naphtyl-2)-6 oxo-6 hexadiène-2,4 oate d'éthyle peut être préparé de la manière suivante :

On opère comme à l'exemple 4, pour la préparation du phényl-6 oxo-6 méthylthio-5 diméthylamino-2 hexadiène-2,4 oate d'éthyle, à partir de 10,6 g de tétrafluoroborate de N-(diméthylamino-3 éthoxycarbonyl-3 propénylidène) N-méthyl méthanaminium, de 18,5 cm3 d'une solution éthanolique 2M d'éthylate de sodium et de 8 g de (méthylthio-2 acétyl)-2 naphtalène dans 50 cm3 d'éthanol. Après purification par chromatographie sur colonne de silice avec un mélange de cyclohexane et d'acétate d' éthyle (50-50 en volumes) comme éluant, on obtient 5 g de diméthylamino-2 méthylthio-5 (naphtyl-2)-6 oxo-6 hexadiène-2,4 oate d'éthyle, sous forme d'une huile orange, utilisé à l'état brut dans les synthèses ultérieures.

Le (méthylthio-2 acétyl)-2 naphtalène peut être préparé de la façon suivante :

On opère comme à l'exemple 4 pour la préparation de l'α-méthylthio acétophénone, à partir de 12,5 g de (bromo-2 acétyl)-2 naphtalène et de 3,5 g de méthanethiolate de sodium dans 100 cm3 d'éthanol. On obtient ainsi 8 g de (méthylthio-2 acétyl)-2 naphtalène utilisé à l'état brut dans les synthèses ultérieures.

EXEMPLE 33

On opère comme à l'exemple 4, à partir de 3,4 g de diméthylamino-2 (phénoxy-3 phényl)-5 hexadiène-2,4 dioate de diéthyle, de 13 cm3 d'une solution aqueuse d'acide chlorhydrique 12N et de 13 cm3 d'éthanol. Le mélange est chauffé 5 minutes à ébullition puis est refroidi à une température d'environ 20° C. Après purification par recristallisation dans 100 cm3 d'oxyde de diisopropyle bouillant, on obtient 2,7 g d'hydroxy-2 (phénoxy-3 phényl)-5 hexadiène-2,4 dioate de diéthyle fondant à 108° C.

Le diméthylamino-2 (phénoxy-3 phényl)-5 hexadiène-2,4 dioate de diéthyle peut être préparé de la manière suivante :

On opère comme à l'exemple 4, pour la préparation du phényl-6 oxo-6 méthylthio-5 diméthylamino-2 hexadiène-2,4 oate d'éthyle, à partir de 7,6 g de tétrafluoroborate de N-(diméthylamino-3 éthoxycarbonyl-3 propénylidène) N-méthyl méthanaminium, de 13,3 cm3 d'une solution éthanolique 2M d'éthylate de sodium et de 6,8 g de (phénoxy-3 phényl) acétate d'éthyle dans 70 cm3 d'éthanol. Après purification par chromatographie sur colonne de silice avec un mélange de cyclohexane et d'acétate d' éthyle (50-50 en volumes) comme éluant, on obtient 3,4 g de diméthylamino-2 (phénoxy-3 phényl)-5 hexadiène-2,4 dioate de diéthyle, sous forme d'une huile jaune, utilisé à l'état brut dans les synthèses ultérieures.

Le (phénoxy-3 phényl) acétate d'éthyle peut être préparé de la manière suivante :

11,5 g d'acide (phénoxy-3 phényl) acétique, 3 cm3 d'une solution aqueuse d'acide sulfurique 36N dans 200 cm3 d'éthanol sont portés à ébullition durant 72 heures. Après refroidissement à environ 20° C, le mélange réactionnel est concentré à sec à 50° C sous pression réduite (20 mm de mercure ; 2,7 kPa). L'huile est dissoute dans 300 cm3 de dichlorométhane ; la phase organique est lavée par 150 cm3 d'eau, séchée sur du sulfate de magnésium anhydre, filtrée et concentrée à sec à 40° C sous pression réduite (20 mm de mercure ;

2,7 kPa). Le résidu est dissous dans 20 cm3 d'un mélange de cyclohexane et d'acétate d'éthyle (50-50 en volumes) et la solution est versée sur 400 g de silice contenus dans une colonne de 5 cm de diamètre. On élue avec un mélange de cyclohexane et d'acétate d'éthyle (50-50 en volumes) et l'éluat correspondant est concentré à sec à 40°C sous pression réduite (20 mm de mercure ; 2,7 kPa). On obtient ainsi 7,4 g de (phénoxy-3 phényl) acétate d'éthyle utilisé à l'état brut dans les synthèses ultérieures.

L'acide (phénoxy-3 phényl) acétique peut être préparé selon la méthode décrite par D.MATTHIES et coll., Arch.Pharm. 604 (1983).

EXEMPLE 34

On opère comme à l'exemple 2, à partir de 7,3 g de diméthylamino-2 (trifluorométhyl-4 benzylthio)-5 hexadiène-2,4 dioate de diéthyle, de 25,5 cm3 d'une solution aqueuse d'acide chlorhydrique 12N et de 50 cm3 d'éthanol. Le mélange est chauffé 1 minute à ébullition puis est refroidi à une température d'environ 20°C. Après purification par recristallisation dans 60 cm3 d'éther de pétrole (40-60°) bouillant, on obtient 4,2 g d'hydroxy-2 (trifluorométhyl-4 benzylthio)-5 hexadiène-2,4 dioate de diéthyle fondant à 73°C.

Le diméthylamino-2 (trifluorométhyl-4 benzylthio)-5 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 5,7 g de tétrafluoroborate de N-(diméthylamino-3 éthoxycarbonyl-3 propénylidène) N-méthyl méthanaminium, de 50 cm3 d'une solution éthanolique 2M d'éthylate de sodium et de 5,6 g de (trifluorométhyl-4 benzylthio) acétate d'éthyle dans 50 cm3 d'éthanol. On obtient ainsi 7,3 g de diméthylamino-2 (trifluorométhyl-4 benzylthio)-5 hexadiène-2,4 dioate de diéthyle, sous forme d'une huile jaune, utilisé à l'état brut dans les synthèses ultérieures.

Le (trifluorométhyl-4 benzylthio) acétate d'éthyle peut être préparé de la façon suivante :

On opère comme à l'exemple 29 pour la préparation du (méthyl-4 benzylthio) acétate d'éthyle, à partir de 9,8 g de chlorométhyl-1 trifluorométhyl-4 benzène, de 25 cm3 d'une solution éthanolique 2M d'éthylate de sodium et de 6,1 g de mercapto-2 acétate d'éthyle dans 120 cm3 d'éthanol. On obtient ainsi 12,2 g de (trifluorométhyl-4 benzylthio) acétate d'éthyle utilisé à l'état brut dans les synthèses ultérieures.

EXEMPLE 35

On opère comme à l'exemple 4, à partir de 9 g de diméthylamino-2 (fluoro-4 phényl)-6 méthylthio-5 oxo-6 hexadiène-2,4 oate d'éthyle, de 36 cm3 d'une solution aqueuse d'acide chlorhydrique 12N et de 100 cm3 d'éthanol. Le mélange est chauffé 5 minutes à ébullition puis est refroidi à une température d'environ 20°C. Après purification par recristallisation dans 50 cm3 de cyclohexane bouillant, on obtient 3,2 g de (fluoro-4 phényl)-6 hydroxy-2 méthylthio-5 oxo-6 hexadiène-2,4 oate d'éthyle fondant à 95°C.

Le diméthylamino-2 (fluoro-4 phényl)-6 méthylthio-5 oxo-6 hexadiène-2,4 oate d'éthyle peut être préparé de la manière suivante :

On opère comme à l'exemple 4, pour la préparation du phényl-6 oxo-6 méthylthio-5 diméthylamino-2 hexadiène-2,4 oate d'éthyle à partir de 10 g de tétrafluoroborate de N-(diméthylamino-3 éthoxycarbonyl-3 propénylidène) N-méthyl méthanaminium, de 21 cm3 d'une solution éthanolique 2M d'éthylate de sodium et de 6,5 g d'α-méthylthio fluoro-4 acétophénone dans 100 cm3 d'éthanol. Après purification par chromatographie sur colonne de silice avec un mélange de cyclohexane et d'acétate d'éthyle (50-50 en volumes) comme éluant, on obtient 9 g de diméthylamino-2 (fluoro-4 phényl)-6 méthylthio-5 oxo-6 hexadiène-2,4 oate d'éthyle, sous forme d'une huile orange, utilisé à l'état brut dans les synthèses ultérieures.

L'α-méthylthio fluoro-4 acétophénone peut être préparée de la façon suivante :

On opère comme à l'exemple 4 pour la préparation de l'α-méthylthio-2 acétophénone, à partir de 25 g d'α-bromo p-fluoro acétophénone et de 10,3 g de méthanethiolate de sodium dans 130 cm3 d'éthanol. Après purification par chromatographie sur colonne de silice avec un mélange de cyclohexane et d'acétate d'éthyle (50-50 en volumes) comme éluant, on obtient 21,9 g d'α-méthylthio fluoro-4 acétophénone utilisée à l'état brut dans les synthèses ultérieures.

EXEMPLE 36

On opère comme à l'exemple 4, à partir de 7,7 g de diméthylamino-2 (bromo-4 phényl)-6 méthylthio-5 oxo-6 hexadiène-2,4 oate d'éthyle, de 25 cm3 d'une solution aqueuse d'acide chlorhydrique 12N et de 80 cm3 d'éthanol. Le mélange est chauffé 5 minutes à ébullition puis est refroidi à une température d'environ 20°C. Après purification par chromatographie sur colonne de silice avec un mélange de cyclohexane et d'acétate d'éthyle (80-20 puis 50-50 en volumes) comme éluant et recristallisation dans 40 cm3 de cyclohexane bouillant, on obtient 2,5 g de (bromo-4 phényl)-6 hydroxy-2 méthylthio-5 oxo-6 hexadiène-2,4 oate d'éthyle fondant à 84°C.

Le diméthylamino-2 (bromo-4 phényl)-6 méthylthio-5 oxo-6 hexadiène-2,4 oate d'éthyle peut être préparé de la manière suivante :

On opère comme à l'exemple 4, pour la réalisation du phényl-6 oxo-6 méthylthio-5 diméthylamino-2 hexadiène-2,4 oate d'éthyle, à partir de 10 g de tétrafluoroborate de N-(diméthylamino-3 éthoxycarbonyl-3 propénylidène) N-méthyl méthanaminium, de 21 cm3 d'une solution éthanolique 2M d'éthylate de sodium et de 8,6 g d'α-méthylthio bromo-4 acétophénone dans 100 cm3 d'éthanol. Après purification par recristallisation dans 100 cm3 de cyclohexane, on obtient 7,7 g de diméthylamino-2 (bromo-4 phényl)-6 méthylthio-5 oxo-6

hexadiène-2,4 oate d'éthyle fondant à 107°C.

L'α-méthylthio bromo-4 acétophénone peut être préparée de la façon suivante :

On opère comme à l'exemple 4 pour la préparation de l'α-méthylthio acétophénone, à partir de 30 g d'α-p-dibromo acétophénone et de 7,6 g de méthanethiolate de sodium dans 100 cm3 d'éthanol. Après purification par chromatographie sur colonne de silice avec un mélange de cyclohexane et de dichlorométhane (50-50 en volumes) comme éluant, on obtient 22,4 g d'α-méthylthio bromo-4 acétophénone utilisée à l'état brut dans les synthèses ultérieures.

EXEMPLE 37

On opère comme à l'exemple 4, à partir de 2,7 g de diméthylamino-2 (biphénylyl-4)-6 méthylthio-5 oxo-6 hexadiène-2,4 oate d'éthyle, de 10,7 cm3 d'une solution aqueuse d'acide chlorhydrique 12N et de 10,7 cm3 d'éthanol. Le mélange est chauffé 5 minutes à ébullition puis est refroidi à une température d'environ 20°C. Après purification par recristallisation dans 100 cm3 d'oxyde de diisopropyle bouillant, on obtient 1,1 g d'hydroxy-2 (biphénylyle-4)-6 méthylthio-5 oxo-6 hexadiène-2,4 oate d'éthyle fondant à 125°C.

Le diméthylamino-2 (biphénylyl-4)-6 méthylthio-5 oxo-6 hexadiène-2,4 oate d'éthyle peut être préparé de la manière suivante :

On opère comme à l'exemple 4, pour la préparation du phényl-6 oxo-6 méthylthio-5 diméthylamino-2 hexadiène-2,4 oate d'éthyle, à partir de 6 g de tétrafluoroborate de N-(diméthylamino-3 éthoxycarbonyl-3 propénylidène) N-méthyl méthanaminium, de 10,5 cm3 d'une solution éthanolique 2M d'éthylate de sodium et de 5,1 g d'α-méthylthio phényl-4 acétophénone dans 150 cm3 d'éthanol. Après purification par chromatographie sur colonne de silice avec un mélange de cyclohexane et d'acétate d'éthyle (50-50 en volumes) comme éluant, on obtient 2,8 g de diméthylamino-2 (biphénylyl-4)-6 méthylthio-5 oxo-6 hexadiène-2,4 oate d'éthyle, sous forme d'une huile orange, utilisé à l'état brut dans les synthèses ultérieures.

L'α-méthylthio phényl-4 acétophénone peut être preparée de la façon suivante :

On opère comme à l'exemple 4 pour la préparation de l'α-méthylthio acétophénone, à partir de 9,6 g d'α-bromo phényl-4 acétophénone et de 2,4 g de méthanethiolate de sodium dans 100 cm3 d'éthanol. On obtient 5,1 g d'α-méthylthio phényl-4 acétophénone, fondant à 92°C, utilisée à l'état brut dans les synthèses ultérieures.

EXEMPLE 38

On opère comme à l'exemple 2, à partir de 22,8 g de diméthylamino-2 phénéthylthio-5 hexadiène-2,4 dioate de diéthyle, de 120 cm3 d'une solution aqueuse d'acide chlorhydrique 12N et de 120 cm3 d'éthanol. Le mélange est chauffé 1 minute à ébullition puis est refroidi à une température d'environ 20°C. Après purification par chromatographie sur colonne de silice avec un mélange de cyclohexane et d'acétate d'éthyle (80-20 en volumes) comme éluant et recristallisation dans 140 cm3 de cyclohexane bouillant, on obtient 13,2 g d'hydroxy-2 phénéthylthio-5 hexadiène-2,4 dioate de diéthyle fondant à 50°C.

Le diméthylamino-2 phénéthylthio-5 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 20 g de tétrafluoroborate de N-(diméthylamino-3 éthoxycarbonyl-3 propénylidène) N-méthyl méthanaminium, de 42 cm3 d'une solution éthanolique 2M d'éthylate de sodium et de 15,7 g de phénéthylthio acétate d'éthyle dans 200 cm3 d'éthanol. On obtient ainsi 22,8 g de diméthylamino-2 phénéthylthio-5 hexadiène-2,4 dioate de diéthyle, sous forme d'une huile orange, utilisé à l'état brut dans les synthèses ultérieures.

Le phénéthylthio acétate d'éthyle peut être préparé de la façon suivante :

On opère comme à l'exemple 29 pour la préparation du (méthyl-4 benzylthio) acétate d'éthyle, à partir de 20 g de (bromo-2 éthyl) benzène, de 25 cm3 d'une solution éthanolique 2M d'éthylate de sodium et de 13 g de mercapto-2 acétate d'éthyle dans 150 cm3 d'éthanol. On obtient ainsi 21,7 g de phénéthylthio acétate d'éthyle utilisé à l'état brut dans les synthèses ultérieures.

EXEMPLE 39

A une solution éthanolique de 2,8 g d'hydroxy-2 phénéthylthio-5 hexadiène-2,4 dioate de diéthyle, on ajoute, en 10 minutes, en maintenant à une température voisine de 20°C, 32 cm3 d'une solution aqueuse de soude 1N. Le mélange est maintenu à une température voisine de 20°C pendant 15 minutes puis est amené à pH 1 à l'aide d'une solution aqueuse d'acide chlorhydrique 1N.

L'éthanol réactionnel est évaporé sous pression réduite (20 mm de mercure ; 2,7 kPa). Après addition de 80 cm3 d'eau, le mélange est extrait par 2 fois 80 cm3 d'acétate d'éthyle. La phase organique est séchée sur sulfate de magnésium anhydre et concentrée à sec à 40°C sous pression réduite (20 mm de mercure ; 2,7 kPa). Le solide ainsi obtenu est recristallisé dans un mélange de cyclohexane et d'acétate d'éthyle (98-2 en volumes) bouillant. On obtient ainsi 1 g d'acide hydroxy-2 éthoxycarbonyl-5 phénéthylthio-5 pentadiène-2,4 oïque fondant à 111°C.

EXEMPLE 40

On opère comme à l'exemple 2, à partir de 11,5 g de diméthylamino-2 (naphtyl-2 thio)-5 hexadiène-2,4 dioate de diéthyle, de 58 cm3 d'une solution aqueuse d'acide chlorhydrique 12N et de 100 cm3 d'éthanol. Le

mélange est chauffé 1 minute à ébullition puis est refroidi à une température d'environ 20° C. Après purification par recristallisation dans 50 cm3 d'éther de pétrole (40-60°) bouillant, on obtient 2 g d'hydroxy-2 (naphtyl-2 thio)-5 hexadiène-2,4 dioate de diéthyle fondant à 90° C.

Le diméthylamino-2 (naphtyl-2 thio)-5 hexadiène-2,4 dioate de diéthyle peut être prépare de la manière suivante :

On opère comme à l'exemple 2 pour la préparation du diméthylamino-2 phénylthio-5 hexadiène-2,4 dioate de diéthyle, à partir de 10 g de tétrafluoroborate de N-(diméthylamino-3 éthoxycarbonyl-3 propénylidène) N-méthyl méthanaminium, de 21 cm3 d'une solution éthanolique 2M d'éthylate de sodium et de 12,9 g de (naphtyl-2 thio) acétate d'éthyle dans 100 cm3 d'éthanol. Après purification par chromatographie sur colonne de silice avec un mélange de cyclohexane et d'acétate d'éthyle (50-50 en volumes) comme éluant, on obtient 11,5 g de diméthylamino-2 (naphtyl-2 thio)-5 hexadiène-2,4 dioate de diéthyle, sous forme d'une huile jaune, utilisé à l'état brut dans les synthèses ultérieures.

Le (naphtyl-2 thio) acétate d'éthyle peut être préparé de la façon suivante :

On opère comme à l'exemple 2 pour la préparation du phénylthio acétate d'éthyle, à partir de 15 g de naphtyl-2 mercaptan, de 48 cm3 d'une solution éthanolique 2M d'éthylate de sodium et de 15,7 g de bromoacétate d'éthyle dans 150 cm3 d'éthanol. On obtient ainsi 16,67 g de (naphtyl-2 thio) acétate d'éthyle utilisé à l'état brut dans les synthèses ultérieures.

EXEMPLE 41

On opère comme à l'exemple 39, à partir de 2 g d'hydroxy-2 (naphtyl-2 thio)-5 hexadiène-2,4 dioate de diéthyle, de 80 cm3 d'une solution aqueuse de soude 1N et de 100 cm3 d'éthanol. On obtient par précipitation dans l'éthanol 1,5 g d'acide hydroxy-2 éthoxycarbonyl-5 (naphtyl-2 thio)-5 pentadiène-2,4 oïque fondant à 182° C.

EXEMPLE 42

On opère comme à l'exemple 2, à partir de 2,2 g de diméthylamino-2 (naphtyl-1)-5 hexadiène-2,4 dioate de diéthyle, de 12 cm3 d'une solution aqueuse d'acide chlorhydrique 12N et de 50 cm3 d'éthanol. Le mélange est chauffé 1 minute à ébullition puis est refroidi à une température d'environ 20° C. Après purification par recristallisation dans 30 cm3 de cyclohexane bouillant, on obtient 0,8 g d'hydroxy-2 (naphtyl-1)-5 hexadiène-2,4 dioate de diéthyle fondant à 99° C.

Le diméthylamino-2 (naphtyl-1)-5 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 10 g de tétrafluoroborate de N-(diméthylamino-3 éthoxycarbonyl-3 propénylidène) N-méthyl méthanaminium, de 26 cm3 d'une solution éthanolique 2M d'éthylate de sodium et de 7,5 g de (naphthyl-1)-2 acétate d'éthyle dans 100 cm3 d'éthanol. Après purification par chromatographie sur colonne de silice avec un mélange de cyclohexane et d'acétate d'éthyle (80-20 en volumes), on obtient 2,2 g de diméthylamino-2 (naphtyl-1)-5 hexadiène-2,4 dioate de diéthyle, sous forme d'une huile jaune, utilisé à l'état brut dans les synthèses ultérieures.

EXEMPLE 43

On opère comme à l'exemple 4, à partir de 5,5 g de diméthylamino-2 (diméthylamino-4 phényl)-6 méthylthio-5 oxo-6 hexadiène-2,4 oate d'éthyle, de 38 cm3 d'une solution aqueuse d'acide chlorhydrique 12N et de 50 cm3 d'éthanol. Le mélange est chauffé 5 minutes à ébullition puis est refroidi à une température d'environ 20° C. Après purification par chromatographie sur colonne de silice avec un mélange de cyclohexane et d'acétate d'éthyle (60-40 en volumes), on obtient 3,9 g de (diméthylamino-4 phényl)-6 hydroxy-2 méthylthio-5 oxo-6 hexadiène-2,4 oate d'éthyle sous forme d'huile brune (Rf=0,2 ; support : gel de silice ; éluant : cyclohexane/acétate d'éthyle 60-40 en volumes).

Le diméthylamino-2 (diméthylamino-4 phényl)-6 méthylthio-5 oxo-6 hexadiène-2,4 oate d'éthyle peut être préparé de la manière suivante :

On opère comme à l'exemple 4, pour la préparation du phényl-6 oxo-6 méthylthio-5 diméthylamino-2 hexadiène-2,4 oate d'éthyle, à partir de 8,6 g de tétrafluoroborate de N-(diméthylamino-3 éthoxycarbonyl-3 propénylidène) N-méthyl méthanaminium, de 15 cm3 d'une solution éthanolique 2M d'éthylate de sodium et de 5,8 g d'α-méthylthio diméthylamino-4 acétophénone dans 60 cm3 d'éthanol. Après purification par chromatographie sur colonne de silice avec un mélange de cyclohexane et d'acétate d' éthyle (50-50 en volumes) comme éluant, on obtient 5,5 g de diméthylamino-2 (diméthylamino-4 phényl)-6 méthylthio-5 oxo-6 hexadiène-2,4 oate d'éthyle, sous forme d'une huile orange, utilisé à l'état brut dans les synthèses ultérieures.

L'α-méthylthio diméthylamino-4 acétophénone peut être preparée de la façon suivante :

On opère comme à l'exemple 4 pour la préparation de l'α-méthylthio acétophénone, à partir de 12,5 g d'α-bromo diméthylamino-4 acétophénone et de 3,6 g de méthanethiolate de sodium dans 65 cm3 d'éthanol. On obtient 9,7 g d'α-méthylthio diméthylamino-4 acétophénone, fondant à 65° C, utilisée à l'état brut dans les synthèses ultérieures.

EXEMPLE 44

On opère comme à l'exemple 4, à partir de 14 g de diméthylamino-2 (benzoyl-3 phényl)-5 hexadiène-2,4 dioate de diéthyle, de 52,3 cm3 d'une solution aqueuse d'acide chlorhydrique 12N et de 52,3 cm3 d'éthanol. Le mélange est chauffé 5 minutes à ébullition puis es refroidi à une température d'environ 20°C. Après purification par chromatographie sur colonne de silice avec un mélange de cyclohexane et d'acétate d'éthyle (50-50 en volumes) comme éluant et recristallisation dans 100 cm3 d'isopropanol bouillant, on obtient 2,3 g d'hydroxy-2 (benzoyl-3 phényl)-5 hexadiène-2,4 dioate de diéthyle fondant à 105°C.

Le diméthylamino-2 (benzoyl-3 phényl)-5 hexadiène-2,4 dioate de diéthyle peut être préparé de la manière suivante :

On opère comme à l'exemple 4, pour la préparation du phényl-6 oxo-6 méthylthio-5 diméthylamino-2 hexadiène-2,4 oate d'éthyle, à partir de 17,8 g de tétrafluoroborate de N-(diméthylamino-3 éthoxycarbonyl-3 propénylidène) N-méthyl méthanaminiun,de 31,3 cm3 d'une solution éthanolique 2M d'éthylate de sodium et de 16,8 g de (benzoyl-3 phényl) acétate d'éthyle dans 100 cm3 d'éthanol. Après purification par chromatographie sur colonne de silice avec du dichlorométhane comme éluant, on obtient 14 g de diméthylamino-2 (benzoyl-3 phényl)-5 hexadiène-2,4 dioate de diéthyle, sous forme d'une huile jaune, utilisé à l'état brut dans les synthèses ultérieures.

Le (benzoyl-3 phényl) acétate d'éthyle peut être préparé de la manière suivante :

On opère comme à l'exemple 33 pour la préparation du (phénoxy-3 phényl) acétate d'éthyle, à partir de 16,6 g d'acide (benzoyl-3 phényl) acétique et de 2 cm3 d'une solution aqueuse d'acide sulfurique 36N dans 200 cm3 d'éthanol. On obtient 16,8 g de (benzoyl-3 phényl) acétate d'éthyle, sous forme d'huile brune, utilisé à l'état brut dans les synthèses ultérieures.

L'acide( benzoyl-3 phényl) acétique peut être préparé selon la méthode décrite par G.COMISSO et coll., Gazz.Chim.Ital. 80, vol.110, 123 (1977).

EXEMPLE 45

On opère comme à l'exemple 4, à partir de 4,2 g de diméthylamino-2 (biphénylyl-3)-5 hexadiène-2,4 dioate de diéthyle, de 17 cm3 d'une solution aqueuse d'acide chlorhydrique 12N et de 17 cm3 d'éthanol. Le mélange est chauffé 5 minutes à ébullition puis est refroidi à une température d'environ 20°C. Après purification par chromatographie sur colonne de silice avec du dichlorométhane comme éluant et recristallisation dans 75 cm3 de cyclohexane bouillant, on obtient 0,7 g de (biphénylyl-3)-5 hydroxy-2 hexadiène-2,4 dioate de diéthyle fondant à 95°C.

Le diméthylamino-2 (biphénylyl-3)-5 hexadiène-2,4 dioate de diéthyle peut être préparé de la manière suivante :

On opère comme à l'exemple 4, pour la préparation du phényl-6 oxo-6 méthylthio-5 diméthylamino-2 hexadiène-2,4 oate d'éthyle, à partir de 8 g de tétrafluoroborate de N-(diméthylamino-3 éthoxycarbonyl-3 propénylidène) N-méthyl méthanaminium, de 14 cm3 d'une solution éthanolique 2M d'éthylate de sodium et de 6,7 g de (biphénylyl-3)-1 acétate d'éthyle dans 30 cm3 d'éthanol. Après purification par chromatographie sur colonne de silice avec du dichlorométhane comme éluant, on obtient 4,2 g de diméthylamino-2 (biphénylyl-3)-5 hexadiène-2,4 dioate de diéthyle, sous forme d'une huile jaune, utilisé à l'état brut dans les synthèses ultérieures.

Le (biphénylyl-3)-1 acétate d'éthyle peut être préparé de la manière suivante :

On opère comme à l'exemple 33 pour la préparation du (phénoxy-3 phényl) acétate d'éthyle, à partir de 6,8 g d'acide (biphénylyl-3) acétique et de 0,5 cm3 d'une solution aqueuse d'acide sulfurique 36N dans 50 cm3 d'éthanol. On obtient 6,8 g de (biphénylyl-3)-1 acétate d'éthyle, sous forme d'huile brune, utilisé à l'état brut dans les synthèses ultérieures.

L'acide (biphénylyl-3) acétique peut être préparé selon la méthode décrite par G. SAM et coll., J. Pharm. Sciences, vol.58, n°8, 953 (1969).

EXEMPLE 46

On opère comme à l'exemple 2, à partir de 9,5 g de diméthylamino-2 (méthyl-2 benzylthio)-5 hexadiène-2,4 dioate de diéthyle, de 50 cm3 d'une solution aqueuse d'acide chlorhydridrique 12N et de 300 cm3 d'éthanol. Le mélange est chauffé 1 minute à ébullition puis est refroidi à une température d'environ 20°C. Après purification par recristallisation dans 100 cm3 de cyclohexane bouillant, on obtient 6,9 g d'hydroxy-2 (méthyl-2 benzylthio)-5 hexadiène-2,4 dioate de diéthyle fondant à 95°C.

Le diméthylamino-2 (méthyl-2 benzylthio)-5 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 10 g de tétrafluoroborate de N-(diméthylamino-3 éthoxycarbonyl-3 propénylidène) N-méthyl méthanaminium, de 21 cm3 d'une solution éthanolique 2M d'éthylate de sodium et de 7,9 g de (méthyl-2 benzylthio) acétate d'éthyle dans 100 cm3 d'éthanol. Après purification par chromatographie sur colonne de silice avec un mélange de cyclohexane et d'acétate d'éthyle (70-30 en volumes) comme éluant, on obtient 9,5 g de diméthylamino-2 (méthyl-2 benzylthio)-5 hexadiène-2,4 dioate de diéthyle, sous forme d'une huile jaune, utilisé à l'état brut dans les synthèses ultérieures.

Le (méthyl-2 benzylthio) acétate d'éthyle peut être préparé de la façon suivante :

On opère comme à l'exemple 29 pour la préparation du (méthyl-4 benzylthio) acétate d'éthyle, à partir de 10

g de bromométhyl-2 toluène, de 30 cm3 d'une solution éthanolique 2M d'éthylate de sodium et de 6,5 g de mercapto-2 acétate d'éthyle dans 100 cm3 d'éthanol. On obtient ainsi 8,9 g de (méthyl-2 benzylthio) acétate d'éthyle utilisé à l'état brut dans les synthèses ultérieures.

EXEMPLE 47

On opère comme à l'exemple 2, à partir de 8,8 g de diméthylamino-2 (isopropyl-4 benzylthio)-5 hexadiène-2,4 dioate de diéthyle, de 44 cm3 d'une solution aqueuse d'acide chlorhydrique 12N et de 100 cm3 d'éthanol. Le mélange est chauffé 1 minute à ébullition puis est refroidi à une température d'environ 20° C. Après purification par recristallisation dans 50 cm3 de cyclohexane bouillant, on obtient 5,6 g d'hydroxy-2 (isopropyl-4 benzylthio)-5 hexadiène-2,4 dioate de diéthyle fondant à 86° C.

Le diméthylamino-2 (isopropyl-4 benzylthio)-5 hexadiène-2,4 dioate de diéthyle peut être préparé de la manière suivante :

On opère comme à l'exemple 2 pour la préparation du diméthylamino-2 phénylthio-5 hexadiène-2,4-dioate de diéthyle, à partir de 10 g de tétrafluoroborate de N-(diméthylamino-3 éthoxycarbonyl-3 propénylidène) N-méthyl méthanaminium, de 21 cm3 d'une solution éthanolique 2M d'éthylate de sodium et de 8,8 g de (isopropyl-4 benzylthio) acétate d'éthyle dans 100 cm3 d'éthanol. Après purification par chromatographie sur colonne de silice avec un mélange de cyclohexane et d'acétate d'éthyle (80-20 en volumes) comme éluant, on obtient 8,8 g de diméthylamino-2 (isopropyl-4 benzylthio)-5 hexadiène-2,4 dioate de diéthyle, sous forme d'une huile orange, utilisé à l'état brut dans les synthèses ultérieures.

L'(isopropyl-4 benzylthio) acétate d'éthyle peut être préparé de la façon suivante :

On opère comme à l'exemple 29 pour la préparation du (méthyl-4 benzylthio) acétate d'éthyle, à partir de 15 g de bromométhyl-1 isopropyl-4 benzène, de 49 cm3 d'une solution éthanolique 2M d'éthylate de sodium et de 9,8 g de mercapto-2 acétate d'éthyle dans 150 cm3 d'éthanol. On obtient ainsi 17,9 g d'(isopropyl-4 benzylthio) acétate d'éthyle utilisé à l'état brut dans les synthèses ultérieures.

EXEMPLE 48

On opère comme à l'exemple 4, à partir de 7,3 g de diméthylamino-2 (chloro-3 phényl)-6 méthylthio-5 oxo-6 hexadiène-2,4 oate d'éthyle, de 30,9 cm3 d'une solution aqueuse d'acide chlorhydrique 12N et de 62 cm3 d'éthanol. Le mélange est chauffé 5 minutes à ébullition puis est refroidi à une température d'environ 20° C. Après purification par recristallisation dans 71 cm3 de cyclohexane bouillant, on obtient 6,6 g de (chloro-3 phényl)-6 hydroxy-2 méthylthio-5 oxo-6 hexadiène-2,4 oate d'éthyle fondant à 90° C.

Le diméthylamino-2 (chloro-3 phényl)-6 méthylthio-5 oxo-6 hexadiène-2,4 dioate de diéthyle peut être préparé de la manière suivante :

On opère comme à l'exemple 4, pour la préparation du phényl-6 oxo-6 méthylthio-5 diméthylamino-2 hexadiène-2,4 oate d'éthyle, à partir de 11,5 g de tétrafluoroborate de N-(diméthylamino-3 éthoxycarbonyl-3 propénylidène) N-méthyl méthanaminium, de 20 cm3 d'une solution éthanolique 2M d'éthylate de sodium et de 8 g d'α-méthylthio chloro-3 acétophénone dans 80 cm3 d'éthanol. Après purification par chromatographie sur colonne de silice avec un mélange de cyclohexane et d'acétate d'éthyle (50-50 en volumes) comme éluant, on obtient 7,3 g de diméthylamino-2 (chloro-3 phényl)-6 méthylthio-5 oxo-6 hexadiène-2,4 oate d'éthyle, sous forme d'une huile orange, utilisé à l'état brut dans les synthèses ultérieures.

L'α-méthylthio chloro-3 acétophénone peut être préparée de la façon suivante :

On opère comme à l'exemple 4 pour la préparation de l'α-méthylthio acétophénone, à partir de 22,3 g d'α-bromo chloro-3 acétophénone et de 6,8 g de méthanethiolate de sodium dans 223 cm3 d'éthanol. On obtient ainsi 17,3 g d'α-méthylthio chloro-3 acétophénone utilisée à l'état brut dans les synthèses ultérieures.

EXEMPLE 49

On opère comme à l'exemple 2, à partir de 11 g de diméthylamino-2 (méthyl-3 benzylthio)-5 hexadiène-2,4 dioate diéthyle, de 58 cm3 d'une solution aqueuse d'acide chlorhydrique 12N et de 100 cm3 d'éthanol. Le mélange est chauffé 1 minute à ébullition puis est refroidi à une température d'environ 20° C. Après purification par chromatographie sur colonne de silice avec un mélange de cyclohexane et d'acétate d'éthyle (80-20 en volumes) comme éluant et recristallisation dans 17 cm3 de cyclohexane bouillant, on obtient 6,6 g d'hydroxy-2 (méthyl-3 benzylthio)-5 hexadiène-2,4 dioate de diéthyle fondant à 78° C.

Le diméthylamino-2 (méthyl-3 benzylthio)-5 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 10 g de tétrafluoroborate de N-(diméthylamino-3 éthoxycarbonyl-3 propénylidène) N-méthyl méthanaminium, de 21 cm3 d'une solution éthanolique 2M d'éthylate de sodium et de 7,9 g de (méthyl-3 benzylthio) acétate d'éthyle dans 100 cm3 d'éthanol. On obtient 11 g de diméthylamino-2 (méthyl-3 benzylthio)-5 hexadiène-2,4 dioate de diéthyle, sous forme d'une huile brune, utilisé à l'état brut dans les synthèses ultérieures.

Le (méthyl-3 benzylthio) acétate d'éthyle peut être préparé de la façon suivante :

On opère comme à l'exemple 29 pour la préparation du (méthyl-4 benzylthio) acétate d'éthyle, à partir de 15 g de bromométhyl-3 toluène, de 44,5 cm3 d'une solution éthanolique 2M d'éthylate de sodium et de 9,7 g de mercapto-2 acétate d'éthyle dans 100 cm3 d'éthanol. On obtient ainsi 14,4 g de (méthyl-3 benzylthio) acétate d'éthyle utilisé à l'état brut dans les synthèses ultérieures.

EXEMPLE 50

On opère comme à l'exemple 2, à partir de 21,9 g de diméthylamino-2 méthylthio-5 hexadiène-2,4 dioate de diéthyle, de 114 cm3 d'une solution aqueuse d'acide chlorhydrique 12N et de 110 cm3 d'éthanol. Le mélange est chauffé 5 minutes à ébullition puis est refroidi à une température d'environ 20° C. Après purification par recristallisation dans 100 cm3 de cyclohexane bouillant, on obtient 9,7 g d'hydroxy-2 méthylthio-5 hexadiène-2,4 dioate de diéthyle fondant à 80° C.

Le diméthylamino-2 méthylthio-5 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 28,6 g de tétrafluoroborate de N-(diméthylamino-3 éthoxycarbonyl-3 propénylidène) N-méthyl méthanaminium, de 50 cm3 d'une solution éthanolique 2M d'éthylate de sodium et de 13,4 g de méthylthio acétate d'éthyle dans 180 cm3 d'éthanol. On obtient ainsi 21,9 g de diméthylamino-2 méthylthio-5 hexadiène-2,4 dioate de diéthyle, sous forme d'une huile brune, utilisé à l'état brut dans les synthèses ultérieures.

Le méthylthio acétate d'éthyle peut être préparé de la façon suivante :

On opère comme à l'exemple 2 pour la préparation du phénylthio acétate d'éthyle, à partir de 7 g de méthane thiolate de sodium et de 16,7 g bromoacétate d'éthyle dans 80 cm3 d'éthanol. On obtient ainsi 13,4 g de méthylthio acétate d'éthyle utilisé à l'état brut dans les synthèses ultérieures.

EXEMPLE 51

On opère comme à l'exemple 2, à partir de 8,5 g de diméthylamino-2 éthylthio-5 hexadiène-2,4 dioate de diéthyle, de 60 cm3 d'une solution aqueuse d'acide chlorhydrique 12N et de 100 cm3 d'éthanol. Le mélange est agité 15 minutes à une température d'environ 20° C. Après purification par chromatographie sur colonne de silice avec un mélange de cyclohexane et d'acétate d'éthyle (80-20 en volumes) comme éluant et recristallisation dans 22 cm3 de cyclohexane bouillant, on obtient 4,4 g d'hydroxy-2 éthylthio-5 hexadiène-2,4 dioate de diéthyle fondant à 65° C.

Le diméthylamino-2 éthylthio-5 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 10 g de tétrafluoroborate de N-(diméthylamino-3 éthoxycarbonyl-3 propénylidène) N-méthyl méthanaminium, de 21 cm3 d'une solution éthanolique 2M d'éthylate de sodium et de 5,2 g d'éthylthio acétate d'éthyle dans 100 cm3 d'éthanol. On obtient ainsi 8,5 g de diméthylamino-2 éthylthio-5 hexadiène-2,4 dioate de diéthyle, sous forme d'une huile rouge, utilisé à l'état brut dans les synthèses ultérieures.

L'éthylthio acétate d'éthyle peut être préparé de la façon suivante :

On opère comme à l'exemple 29 pour la préparation du (méthyl-4 benzylthio) acétate d'éthyle, à partir de 10,5 g de iodoéthane, de 37 cm3 d'une solution éthanolique 2M d'éthylate de sodium et de 8,1 g de mercapto-2 acétate d'éthyle dans 100 cm3 d'éthanol. On obtient ainsi 6,9 g d'éthylthio acétate d'éthyle utilisé à l'état brut dans les synthèses ultérieures.

EXEMPLE 52

On opère comme à l'exemple 2 , à partir de 4,7g de diméthylamino-2 phénylthio-5 hexadiène-2,4 dioate de (6) éthyle et de (1) tert-butyle, de 25 cm3 d'une solution aqueuse d'acide chlorhydrique 12N et de 50 cm3 d'éthanol. Le mélange est chauffé 1 minute à ébullition puis est refroidi à une température d'environ 20° C. Après purification par recristallisation dans 88 cm3 d'un mélange d'éther de pétrole (40-60° ) et d'éther de diisopropyle bouillant (50-50 en volumes), on obtient 3 g d'hydroxy-2 phénylthio-5 hexadiène-2,4 dioate de (6) éthyle et de (1) tert-butyle fondant à 113° C.

Le diméthylamino-2 phénylthio-5 hexadiène-2,4 dioate de (6) éthyle et de (1) tert-butyle peut être préparé de la manière suivante :

On opère comme à l'exemple 2 pour la préparation du diméthylamino-2 phénylthio-5 hexadiène-2,4 dioate de diéthyle, à partir de 4,05 g de tétrafluoroborate de N-(diméthylamino-3 t-butyloxycarbonyl-3 propénylidène) N-méthyl méthanaminium, de 8,8 cm3 d'une solution 1,6M de butyllithium dans de l'hexane et de 2,8 g de phénylthioacétate d'éthyle dans 65 cm3 de tétrahydrofuranne. Après purification par chromatographie sur colonne de silice avec un mélange de cyclohexane et d'acétate d'éthyle (70-30 en volumes) comme éluant, on obtient 2,6 g de diméthylamino-2 phénylthio-5 hexadiène-2,4 dioate de (6) éthyle et de (1) tert-butyle fondant à 85-88° C.

Le tétrafluoroborate de N-(diméthylamino-3 t-butyloxycarbonyl-3 propénylidène) N-méthyl méthanaminium peut être obtenu de la façon suivante :

On opère comme à l'exemple 2 pour la préparation du tétrafluoroborate de N-(diméthylamino-3 éthoxycarbonyl-3 propénylidène) N-méthyl méthanaminium, à partir de 5,2 g de tétrafluoroborate de N-(éthoxy-3 t-butyloxycarbonyl-3 propénylidène) N-méthyl méthanaminium et de 1,1 cm3 de diméthylamine en solution dans 8 cm3 de dichlorométhane. Après séparation par filtration, on obtient 4,1 g de tétrafluoroborate de N-(diméthylamino-3 t-butyloxycarbonyl-3 propénylidène) N-méthyl méthanaminium fondant à 137° C.

Le tétrafluoroborate de N-(éthoxy-3 t-butyloxycarbonyl-3 propénylidène) N-méthyl méthanaminium peut être obtenu de la façon suivante :

On opère comme à l'exemple 2 pour la préparation du tétrafluoroborate de N-(éthoxy-3 éthoxycarbonyl-3

propénylidène) N-méthyl méthanaminium, à partir de 3,3 g de diméthylamino-4 oxo-2 butène-3 oate de t-butyle et de 3,62 g de tétrafluoroborate de triéthyloxonium dans 20 cm3 de dichlorométhane. La solution chlorométhylénique de tétrafluoroborate de N-(éthoxy-3 t-butyloxycarbonyl-3 propénylidène) N-méthyl méthanaminium ainsi obtenue est conservée sous une atmosphère d'argon et est utilisée immédiatement dans les synthèses ultérieures.

Le diméthylamino-4 oxo-2 butène-3 oate de t-butyle peut être obtenue de la façon suivante :

On opère comme à l'exemple 2 pour la préparation du diméthylamino-4 oxo-2 butène-3 oate d'éthyle, à partir de 9,7 g de pyruvate de t-butyle et de 11,4 g d'acétal diéthylique de N,N-diméthylformamide. Après purification par chromatographie sur colonne de silice avec de l'acétate d'éthyle comme éluant et recristallisation dans 50 cm3 d'éther de diisopropyle bouillant, on obtient 3,3 g de diméthylamino-4 oxo-2 butène-3 oate de t-butyle fondant à 93°C .

Le pyruvate de t-butyle peut être préparé selon la méthode décrite par H.C. BROWN et coll., J. ORG. CHEM., 50, 1384 (1950).

EXEMPLE 53

On opère comme à l'exemple 2, à partir de 10 g de diméthylamino-2 n-butylthio-5 hexadiène-2,4 dioate de diéthyle, de 63 cm3 d'une solution aqueuse d'acide chlorhydrique 12N et de 100 cm3 d'éthanol. Le mélange est maintenu 1 heure à une température d'environ de 20°C. Après purification par chromatographie sur colonne de silice avec du dichlorométhane comme éluant, on obtient 5,6 g d'hydroxy-2 n-butylthio-5 hexadiène-2,4 dioate de diéthyle fondant à 37°C.

Le diméthylamino-2 n-butylthio-5 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 10 g de tétrafluoroborate de N-(diméthylamino-3 éthoxycarbonyl-3 propénylidène) N-méthyl méthanaminium, de 17,5 cm3 d'une solution éthanolique 2M d'éthylate de sodium et de 6,16 g de n-butylthio acétate d'éthyle dans 60 cm3 d'éthanol. On obtient ainsi 10 g de diméthylamino-2 n-butylthio-5 hexadiène-2,4 dioate de diéthyle, sous forme d'une huile rouge orangée, utilisé a l'état brut dans les synthèses ultérieures.

Le n-butylthio acétate d'éthyle peut être préparé de la façon suivante :

On opère comme à l'exemple 2 pour la préparation du phénylthioacétate d'éthyle, à partir de 10,7 cm3 de n-butylmercaptan, de 50 cm3 d'une solution éthanolique 2M d'éthylate de sodium et de 16,7 g de bromoacétate d'éthyle dans 100 cm3 d'éthanol. On obtient ainsi 15,4 g de n-butylthio acétate d'éthyle utilisé à l'état brut dans les synthèses ultérieures.

EXEMPLE 54

On opère comme à l'exemple 2, à partir de 9,8 g de diméthylamino-2 isopropylthio-5 hexadiène-2,4 dioate de diéthyle, de 63 cm3 d'une solution aqueuse d'acide chlorhydrique 12N et de 98 cm3 d'éthanol. Le mélange est agité 1 heure à une température d'environ 20°C. Après purification par recristallisation dans 90 cm3 de pentane bouillant, on obtient 5,3 g d'hydroxy-2 isopropylthio-5 hexadiène-2,4 dioate de diéthyle fondant à 68°C.

Le diméthylamino-2 isopropylthio-5 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 10 g de tétrafluoroborate de N-(diméthylamino-3 éthoxycarbonyl-3 propénylidène) N-méthyl méthanaminium, de 17,5 cm3 d'une solution éthanolique 2M d'éthylate de sodium et de 5,7 g d'isopropylthio acétate d'éthyle dans 57 cm3 d'éthanol. On obtient ainsi 9,8 g de diméthylamino-2 isopropylthio-5 hexadiène-2,4 dioate de diéthyle, sous forme d'une huile orangée, utilisé à l'état brut dans les synthèses ultérieures.

L'isopropylthio acétate d'éthyle peut être préparé de la façon suivante : On opère comme à l'exemple 2 pour la préparation du phénylthioacétate d'éthyle, à partir de 9,3 cm3 d'isopropylmercaptan, de 50 cm3 d'une solution éthanolique 2M d'éthylate de sodium et de 11,1 cm3 de bromoacétate d'éthyle dans 100 cm3 d'éthanol. On obtient ainsi 14 g d'isopropylthio acétate d'éthyle utilisé à l'état brut dans les synthèses ultérieures.

EXEMPLE 55

On opère comme à l'exemple 2, à partir de 4,5 g de diméthylamino-5 phényl-5 phénéthylthio-2 pentadiène-2,4 oate d'éthyle, de 23,6 cm3 d'une solution aqueuse d'acide chlorhydrique 12N et de 45 cm3 d'éthanol. Le mélange est agité 3 heures à une température d'environ 20°C. Après purification par chromatographie sur colonne de silice avec du dichlorométhane comme éluant, on obtient 2,8 g d'oxo-5 phényl-5 phénéthylthio-2 pentène-2 oate d'éthyle sous forme d'une huile jaune (Rf=0,4 ; support : gel de silice ; éluant : dichlorométhane).

Le diméthylamino-5 phényl-5 phénéthylthio-2 pentadiène-2,4 oate d'éthyle peut être préparé de la manière suivante :

On opère comme à l'exemple 24 pour la préparation du diméthylamino-5 phényl-5 phénylthio-2 pentadiène-2,4 oate d'éthyle, à partir de 5,8 g de tétrafluoroborate de N-(diméthylamino-3 phényl-3 propénylidène) N-méthyl méthanaminium, de 10 cm3 d'une solution éthanolique 2M d'éthylate de sodium et de

4,5 g de phénéthylthio acétate d'éthyle dans 50 cm3 d'éthanol. Après purification par chromatographie sur colonne de silice avec du dichlorométhane comme éluant, on obtient 4,5 g de diméthylamino-5 phényl-5 phénéthylthio-2 pentadiène-2,4 oate d'éthyle, sous forme d'une huile jaune, utilisé à l'état brut dans les synthéses ultérieures.

EXEMPLE 56

On opère comme à l'exemple 2, à partir de 4 g de diméthylamino-5 benzoyl-5 phénylthio-2 pentadiène-2,4 oate d'éthyle, de 21 cm3 d'une solution aqueuse d'acide chlorhydrique 12N et de 40 cm3 d'éthanol. Le mélange est chauffé 3 minutes à ébullition puis est refroidi à une température d'environ 20°C. Après purification par recristallisation dans 70 cm3 de cyclohexane bouillant, on obtient 2,9 g d'hydroxy-5 oxo-6 phényl-6 phénylthio-2 hexadiène-2,4 oate d'éthyle fondant à 110°C.

Le diméthylamino-5 benzoyl-5 phénylthio-2 pentadiène-2,4 oate d'éthyle peut être préparé de la manière suivante :

On opère comme à l'exemple 2 pour la préparation du diméthylamino-2 phénylthio-5 hexadiène-2,4 dioate de diéthyle, à partir de 5 g de tétrafluoroborate de N-(diméthylamino-3 benzoyl-3 propénylidène) N-méthyl méthanaminium, de 7,9 cm3 d'une solution éthanolique 2M d'éthylate de sodium et de 3,1 g de phénylthioacétate d'éthyle dans 50 cm3 d'éthanol. Après purification par chromatographie sur colonne de silice avec un mélange de cyclohexane et d'acétate d'éthyle (80-20 en volumes) comme éluant, on obtient 4,1 g de diméthylamino-5 benzoyl-5 phénylthio-2 pentadiène-2,4 oate d'éthyle fondant à 58°C.

Le tétrafluoroborate de N-(diméthylamino-3 benzoyl-3 propénylidène) N-méthyl méthanaminium peut être préparé de la façon suivante :

On opère comme à l'exemple 24 pour la préparation du tétrafluoroborate de N-(diméthylamino-3 phényl-3 propénylidène) N-méthyl méthanaminium, à partir de 9,3 g de diméthylamino-4 phényl-1 butène-3 dione-1,2, de 10,3 g de tétrafluoroborate de triéthyloxonium et de 3,2 cm3 de diméthylamine dans 140 cm3 de dichlorométhane. Après précipitation dans 30 cm3 d'acétate d'éthyle, on obtient 5 g de tétrafluoroborate de N-(diméthylamino-3 benzoyl-3 propénylidène) N-méthyl méthanaminium fondant à 140°C.

La diméthylamino-4 phényl-1 butène-3 dione-1,2 peut être préparée de la façon suivante :

On opère comme à l'exemple 2 pour la préparation du diméthylamino-4 oxo-2 butène-3 oate d'éthyle, à partir de 25 g de phényl-1 propanedione-1,2 et de 28,3 g d'acétal diéthylique de N,N-diméthylformamide. Après purification par chromatographie sur colonne de silice avec de l'acétate d'éthyle comme éluant, on obtient 9,3 g de (diméthylamino-2 éthényl)-1 phényl-3 propanedione-1,2 fondant à 88°C.

EXEMPLE 57

On opère comme à l'exemple 2, à partir de 6,4 g de diméthylamino-2 (triméthyl-2,4,6 benzylthio)-5 hexadiène-2,4 dioate de diéthyle, de 32 cm3 d'une solution aqueuse d'acide chlorhydrique 12N et de 80 cm3 d'éthanol. Le mélange est agité 90 minutes à une température d'environ 20°C. Après purification par recristallisation dans 100 cm3 de cyclohexane bouillant, on obtient 4,28 g d'hydroxy-2 (triméthyl-2,4,6 benzylthio)-5 hexadiène-2,4 dioate de diéthyle fondant à 122°C .

Le diméthylamino-2 (triméthyl-2,4,6-benzylthio)-5 hexadiène-2,4 dioate diéthyle peut être préparé de la manière suivante :

On opère comme à l'exemple 2 pour la préparation du diméthylamino-2 phénylthio-5 hexadiène-2,4 dioate de diéthyle, à partir de 10 g de tétrafluoroborate de N-(diméthylamino-3 éthoxycarbonyl-3 propénylidène) N-méthyl méthanaminium, de 21 cm3 d'une solution éthanolique 2M d'éthylate de sodium et de 8,8 g de (triméthyl-2,4,6 benzylthio) acétate d'éthyle dans 80 cm3 d'éthanol. Après purification par chromatographie sur colonne de silice avec un mélange de cyclohexane et d'acétate d'éthyle (70-30 en volumes) comme éluant, on obtient 6,44 g de diméthylamino-2 (triméthyl-2,4,6 benzylthio)-5 hexadiène-2,4 dioate de diéthyle, sous forme d'une huile jaune, utilisé à l'état brut dans les synthèses ultérieures.

Le (triméthyl-2,4,6 benzylthio) acétate d'éthyle peut être préparé de la façon suivante :

On opère comme à l'exemple 29 pour la préparation du (méthyl-4 benzylthio) acétate d'éthyle, à partir de 10,25 g de chlorure de triméthyl-2,4,6 benzyle, de 32,5 cm3 d'une solution éthanolique 2M d'éthylate de sodium et de 7,1 g de mercapto-2 acétate d'éthyle dans 150 cm3 d'éthanol. On obtient ainsi 11 g de (triméthyl-2,4,6 benzylthio) acétate d'éthyle utilisé à l'état brut dans les synthèses ultérieures.

EXEMPLE 58

On opère comme à l'exemple 2, à partir de 9 g de diméthylamino-2 (naphtyl-2 méthanethio)-5 hexadiène-2,4 dioate de diéthyle, de 42 cm3 d'une solution aqueuse d'acide chlorhydrique 12N et de 100 cm3 d'éthanol. Le mélange est chauffé 1 minute à ébullition puis est refroidi à une température d'environ 20°C. Après purification par recristallisation dans 100 cm3 de cyclohexane bouillant, on obtient 7 g d'hydroxy-2 (naphtyl-2 méthanethio)-5 hexadiène-2,4 dioate de diéthyle fondant à 88°C.

Le diméthylamino-2 (naphtyl-2 méthanethio)-5 hexadiène-2,4 dioate de diéthyle peut être préparé de la manière suivante :

On opère comme à l'exemple 2 pour la préparation du diméthylamino-2 phénylthio-5 hexadiène-2,4 dioate de diéthyle, à partir de 10 g de tétrafluoroborate de N-(diméthylamino-3 éthoxycarbonyl-3 propénylidène) N-méthyl méthanaminium, de 21 cm3 d'une solution éthanolique 2M d'éthylate de sodium et de 9,1 g de (naphtyl-2 méthanethio) acétate d'éthyle dans 100 cm3 d'éthanol. Après purification par chromatographie sur

colonne de silice avec un mélange de cyclohexane et d'acétate d'éthyle (80-20 en volumes) comme éluant, on obtient 9 g de diméthylamino-2 (naphtyl-2 méthanethio)-5 hexadiène-2,4 dioate de diéthyle, sous forme d'une huile jaune, utilisé à l'état brut dans les synthèses ultérieures.

Le (naphtyl-2 méthanethio) acétate d'éthyle peut être préparé de la façon suivante :

On opère comme à l'exemple 29 pour la préparation du (méthyl-4 benzylthio) acétate d'éthyle, à partir de 20 g de bromométhyl-2 naphtalène, de 5,1 g d'hydrure de sodium à 50 % en dispersion dans l'huile de vaseline et de 10,9 g de mercapto-2 acétate d'éthyle dans 200 cm3 de tétrahydrofuranne. Après purification par chromatographie sur colonne de silice avec un mélange de cyclohexane et d'acétate d'éthyle (80-20 en volumes) comme éluant, on obtient 12 g de (naphtyl-2 méthylthio) acétate d'éthyle utilisé à l'état brut dans les synthèses ultérieures.

Les médicaments selon l'invention sont constitués par un composé de formule (I) à l'état pur ou sous forme d'une composition dans laquelle il est associé à tout autre produit pharmaceutiquement compatible, pouvant être inerte ou physiologiquement actif. Ces médicaments selon l'invention peuvent être utilisés par voie orale, parentérale, rectale ou topique.

Comme compositions solides pour administration orale, peuvent être utilisés des comprimés, pilules, poudres (capsules de gélatine, cachets) ou granulés. Dans ces compositions, le principe actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutique humaine, les composés selon l'invention sont particulièrement utiles comme antiinflammatoires, comme agents protecteurs notamment sur le tractus gastroinstestinal, et pour le traitement de l'asthme, des maladies allergiques, du psoriasis, de l'arthrite rhumatoïde et des fibroses notamment des fibroses hépathiques.

Les doses dépendent de l'effet recherché, de la durée du traitement et de la voie d'administration utilisée ; elles sont généralement comprises entre 0,1 g et 5 g par jour par voie orale pour un adulte avec des doses unitaires allant de 20 à 200 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

EXEMPLE A

On prépare, selon la technique habituelle, des gélules dosées à 50 mg de produit actif ayant la composition suivante :

- hydroxy-2 méthylthio-5 oxo-6 (pipéridino-4 phényl)-6 hexadiène-2,4 oate d'éthyle 50 mg
- cellulose 18 mg
- lactose 55 mg
- silice colloïdale 1 mg
- carboxyméthylamidon sodique 10 mg
- talc 10 mg
- stéarate de magnésium 1 mg

EXEMPLE B

On prépare, selon la technique habituelle, des comprimés dosés à 50 mg de produit actif ayant la composition suivante :

- hydroxy-5 phenylthio-2 pentadiène-2,4 oate d'éthyle 50 mg
- lactose 104 mg
- cellulose 40 mg
- polyvidone 10 mg
- carboxyméthylamidon sodique 22 mg
- talc 10 mg
- stéarate de magnésium 2 mg
- silice colloïdale 2 mg
- mélange d'hydroxyméthylcellulose, glycérine, oxyde de titane (72-3,5-24,5) q. s. p. 1 comprimé pelliculé terminé à 245 mg

EXEMPLE C

On prépare une solution injectable contenant 10 mg de produit actif ayant la composition suivante :
- acide hydroxy-2 phénylthio-5 éthoxycarbonyl-5 pentadiène-2,4 oïque 10 mg
- acide benzoïque 80 mg
- alcool benzylique 0,06 cm3
- benzoate de sodium 80 mg
- éthanol à 95 % 0,4 cm3
- hydroxyde de sodium 24 mg
- propylène glycol 1,6 cm3
- eau q. s. p. 4 cm3

## Revendications

1 - Les composés de formule :

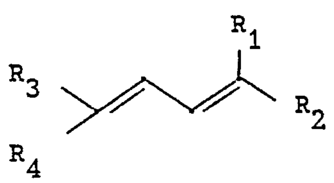

(I)

dans laquelle, $R_1$ représente un radical hydroxy ou acétoxy, $R_2$ représente un atome d'hydrogène, un radical carboxy, alcoxycarbonyle, phényle ou benzoyle et
- soit $R_3$ représente un radical alkylthio ou alcoxy et $R_4$ représente un radical naphtoyle, benzoyle ou benzoyle substitué par un ou plusieurs atomes d'halogène, un ou plusieurs radicaux alkyle, alcoxy, phényle, phénoxy, pipéridino, diméthylamino ou hydroxy ou en positions -3 et -4 par un radical isopropylènedioxy,
- soit $R_3$ représente un radical alcoxycarbonyle, cycloalkyloxycarbonyle dont la partie cycloalkyle comporte 3 à 6 atomes de carbone ou cyano et $R_4$ représente un radical alkyle (1-8C), naphtyle, phényle éventuellement substitué (par un radical phénoxy, phényle, naphtyle ou benzoyle), alkylthio, naphtylméthanethio, benzylthio éventuellement substitué (par un ou plusieurs radicaux alkyle ou un radical trifluorométhyle, phényle ou phénoxy), phénylthio éventuellement substitué (par un atome d'halogène ou un radical alcoxy), naphtylthio, phénéthylthio ou allylthio,
- soit $R_3$ et $R_4$ forment ensemble avec l'atome de carbone auquel ils sont rattachés un des cycles de formules :

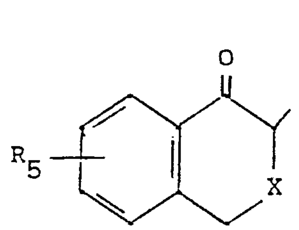

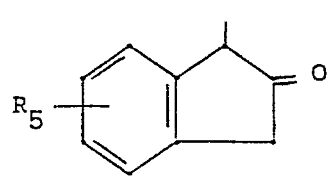

dans lesquelles $R_5$ représente un atome d'hydrogène ou un radical alcoxy et X représente un radical

méthylène ou un atome de soufre, étant entendu que lorsque $R_1$ représente un radical acétoxy, $R_4$ ne peut pas représenter un radical benzoyle substitué par un ou plusieurs radicaux hydroxy, que, sauf mention contraire, dans les définitions qui précèdent les radicaux alkyle et alcoxy, et les portions alkyle et alcoxy contiennent 1 à 4 atomes de carbone en chaîne droite ou ramifiée ainsi que la forme tautomère de ces composés lorsque $R_1$ représente un radical hydroxy.

2 - Procédé de préparation d'un composé selon la revendication 1 dans la formule duquel $R_1$ représente le radical hydroxy, $R_2$ représente un atome d'hydrogène ou un radical alcoxycarbonyle, phényle ou benzoyle et $R_3$ et $R_4$ sont définis comme dans la revendication 1 caractérisé en ce que l'on hydrolyse en milieu acide une énamine de formule :

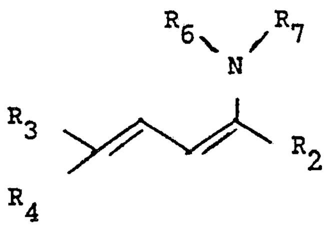

(II)

dans laquelle $R_2$, $R_3$ et $R_4$ ont les significations mentionnées ci-dessus et $R_6$ et $R_7$ représentent des radicaux alkyle ou $R_6$ et $R_7$ forment ensemble avec l'atome d'azote auquel ils sont rattachés un hétérocycle puis isole le produit obtenu.

3 - Les énamines de formule :

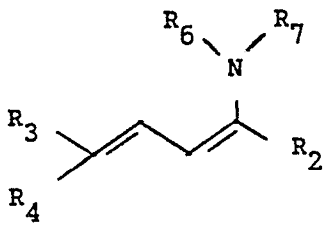

(II)

dans laquelle $R_2$ représente un atome d'hydrogène ou un radical alcoxycarbonyle, phényle ou benzoyle et
- soit $R_3$ représente un radical alkylthio ou alcoxy et $R_4$ représente un radical naphtoyle, benzoyle ou benzoyle substitué par un ou plusieurs atomes d'halogène, un ou plusieurs radicaux alkyle, alcoxy, phényle, phénoxy, pipéridino, diméthylamino ou hydroxy ou en positions -3 et -4 par un radical isopropylènedioxy,
- soit $R_3$ représente un radical alcoxycarbonyle, cycloalkyloxycarbonyle dont la partie cycloalkyle comporte 3 à 6 atomes de carbone ou cyano et $R_4$ représente un radical alkyle (1-8C), naphtyle, phényle éventuellement substitué (par un radical phénoxy, phényle, naphtyle ou benzoyle), alkylthio, napthylméthanethio, benzylthio éventuellement substitué (par un ou plusieurs radicaux alkyle ou un radical trifluorométhyle, phényle ou phénoxy), phénylthio éventuellement substitué (par un atome d'halogène ou un radical alcoxy), naphtylthio, phénéthylthio ou allylthio,
- soit $R_3$ et $R_4$ forment ensemble avec l'atome de carbone auquel ils sont rattachés un des cycles de formules :

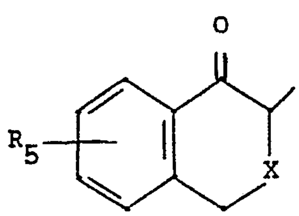

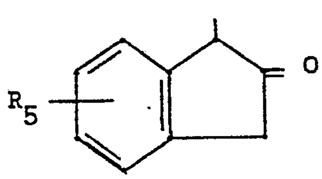

dans lesquelles $R_5$ représente un atome d'hydrogène ou un radical alcoxy et X représente un radical méthylène ou un atome de soufre, $R_6$ et $R_7$ représentent des radicaux alkyle ou $R_6$ et $R_7$ forment

ensemble avec l'atome d'azote auquel ils sont rattachés un hétérocycle, étant entendu que, sauf mention contraire, dans les définitions qui précèdent, les radicaux alkyle et alcoxy et les portions alkyle et alcoxy contiennent 1 à 4 atomes de carbone en chaîne droite ou ramifiée et à l'exception du diméthylamino-2 cyano-5 phényl-5 pentadiène-2,4 oate d'éthyle.

4 - Procédé de préparation d'un composé selon la revendication 1 dans la formule duquel $R_1$ représente un radical hydroxy, $R_2$ représente un radical alcoxycarbonyle, $R_3$ et $R_4$ sont définis comme dans la revendication 1 caractérisé en ce que l'on hydrolyse en milieu acide une énamine de formule :

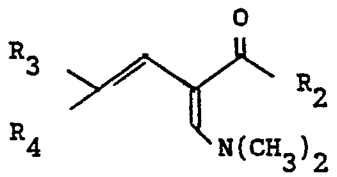
(VI)

dans laquele $R_2$, $R_3$ et $R_4$ ont les significations mentionnées ci-dessus puis isole le produit.

5 - Les énamines de formule :

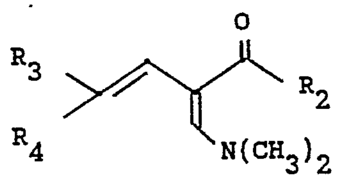
(VI)

dans laquelle $R_2$ représente un radical alcoxycarbonyle et
- soit $R_3$ représente un radical alkylthio ou alcoxy et $R_4$ représente un radical naphtoyle, benzoyle ou benzoyle substitué par un ou plusieurs atomes d'halogène, un ou plusieurs radicaux alkyle, alcoxy, phényle, phénoxy, pipéridino, diméthylamino ou hydroxy ou en positions -3 et -4 par un radical isopropylènedioxy,
- soit $R_3$ représente un radical alcoxycarbonyle, cycloalkyloxycarbonyle dont la partie cycloalkyle comporte 3 à 6 atomes de carbone ou cyano et $R_4$ représente un radical alkyle (1-8C), naphtyle, phényle éventuellement substitué (par un radical phénoxy, phényle, naphtyle ou benzoyle), alkylthio, napthylméthanethio, benzylthio éventuellement substitué (par un ou plusieurs radicaux alkyle ou un radical trifluorométhyle, phényle ou phénoxy), phénylthio éventuellement substitué (par un atome d'halogène ou un radical alcoxy), naphtylthio, phénéthylthio ou allylthio,
- soit $R_3$ et $R_4$ forment ensemble avec l'atome de carbone auquel ils sont rattachés un des cycles de formules :

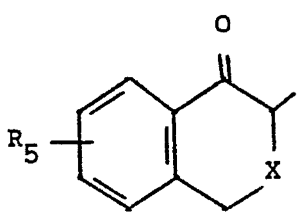

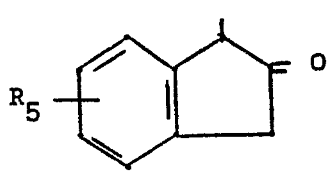

dans lesquelles $R_5$ représente un atome d'hydrogène ou un radical alcoxy et X représente un radical méthylène ou un atome de soufre,

6 - Procédé de préparation d'un composé selon la revendication 1 dans la formule duquel $R_1$ représente le radical hydroxy, $R_2$ représente le radical carboxy, $R_3$ et $R_4$ sont définis comme dans la revendication 1 caractérisé en ce que l'on saponifie l'ester énol de formule :

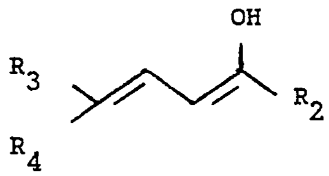

(VII)

dans laquelle $R_2$ représente un radical alcoxycarbonyle et $R_3$, $R_4$ sont définis comme dans la revendication 1 et isole le produit.

7 - Procédé de préparation d'un composé selon la revendication 1 dans la formule duquel $R_1$ représente le radical acétoxy et $R_2$, $R_3$ et $R_4$ sont définis comme dans la revendication 1 étant entendu que $R_3$ ne peut pas représenter un radical benzoyle substitué par un ou plusieurs radicaux hydroxy caractérisé en ce que l'on fait réagir le chlorure d'acétyle sur le composé de formule (I) correspondant dans laquelle $R_1$ représente le radical hydroxy et isole le produit.

8 - Médicaments contenant au moins une substance active et éventuellement un ou plusieurs diluants ou adjuvants compatibles et pharmaceutiquement acceptables caractérisés en ce que la substance active est un composé selon la revendication 1.

**Revendications pour les Etats contractants suivants : GR, ES**

1 - Procédé de préparation des composés de formule :

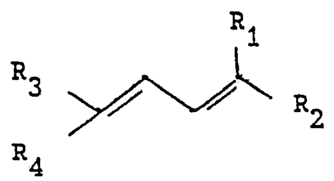

(I)

dans laquelle, $R_1$ représente un radical hydroxy ou acétoxy, $R_2$ représente un atome d'hydrogène, un radical carboxy, alcoxycarbonyle, phényle ou benzoyle et

- soit $R_3$ représente un radical alkylthio ou alcoxy et $R_4$ représente un radical naphtoyle, benzoyle ou benzoyle substitué par un ou plusieurs atomes d'halogène, un ou plusieurs radicaux alkyle, alcoxy, phényle, phénoxy, pipéridino, diméthylamino ou hydroxy ou en positions -3 et -4 par un radical isopropylènedioxy,
- soit $R_3$ représente un radical alcoxycarbonyle, cycloalkyloxycarbonyle dont la partie cycloalkyle comporte 3 à 6 atomes de carbone ou cyano et $R_4$ représente un radical alkyle (1-8C), naphtyle, phényle éventuellement substitué (par un radical phénoxy, phényle, naphtyle ou benzoyle), alkylthio, naphtylméthanethio, benzylthio éventuellement substitué (par un ou plusieurs radicaux alkyle ou un radical trifluorométhyle, phényle ou phénoxy), phénylthio éventuellement substitué (par un atome d'halogène ou un radical alcoxy), naphtylthio, phénéthylthio ou allylthio,
- soit $R_3$ et $R_4$ forment ensemble avec l'atome de carbone auquel ils sont rattachés un des cycles de formules :

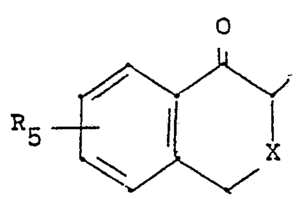

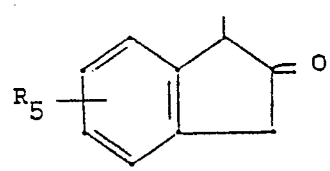

dans lesquelles $R_5$ représente un atome d'hydrogène ou un radical alcoxy et X représente un radical méthylène ou un atome de soufre, étant entendu que lorsque $R_1$ représente un radical acétoxy, $R_4$ ne peut pas représenter un radical benzoyle substitué par un ou plusieurs radicaux hydroxy, que, sauf mention contraire, dans les définitions qui précèdent et qui suivent les radicaux alkyle et alcoxy, et les portions alkyle et alcoxy contiennent 1 à 4 atomes de carbone en chaîne droite ou ramifiée ainsi que la

forme tautomère de ces composés lorsque $R_1$ représente un radical hydroxy, caractérisé en ce que :

A - Pour la préparation d'un composé de formule (I) dans laquelle $R_1$ représente le radical hydroxy, $R_2$ représente un atome d'hydrogène ou un radical alcoxycarbonyle, phényle ou benzoyle et $R_3$ et $R_4$ sont définis comme précédemment, on hydrolyse en milieu acide une énamine de formule :

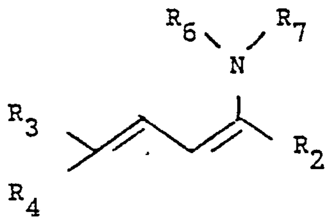

(II)

dans laquelle $R_2$, $R_3$ et $R_4$ sont définis comme précédemment et $R_6$ et $R_7$ représentent des radicaux alkyle ou $R_6$ et $R_7$ forment ensemble avec l'atome d'azote auquel ils sont rattachés un hétérocycle puis isole le produit obtenu.

B - Pour la préparation d'un composé de formule (I) dans laquelle $R_1$ représente le radical hydroxy, $R_2$ représente un radical alcoxycarbonyle, $R_3$ et $R_4$ sont définis comme précédemment, on hydrolyse en milieu acide une énamine de formule :

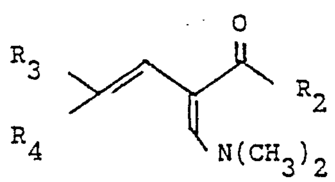

(VI)

dans laquelle $R_2$, $R_3$ et $R_4$ sont définis comme précédemment puis isole le produit obtenu.

C - Pour la préparation d'un composé de formule (I) dans laquelle $R_1$ représente le radical hydroxy, $R_2$ représente le radical carboxy, $R_3$ et $R_4$ sont définis comme précédemment, on saponifie l'ester énol de formule :

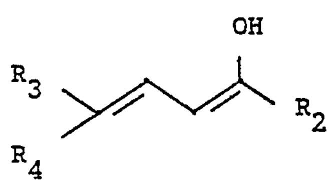

(VII)

dans laquelle $R_2$ représente un radical alcoxycarbonyle et $R_3$, $R_4$ sont définis comme précédemment et isole le produit obtenu.

D - Pour la préparation d'un composé de formule (I) dans laquelle $R_1$ représente le radical acétoxy et $R_2$, $R_3$ et $R_4$ sont définis comme précédemment, étant entendu que $R_3$ ne peut pas représenter un radical benzoyle substitué par un ou plusieurs radicaux hydroxy, on fait réagir le chlorure d'acétyle sur le composé de formule (I) correspondant pour lequel $R_1$ représente le radical hydroxy et isole le produit obtenu.

2 - Procédé de préparation des énamines de formule :

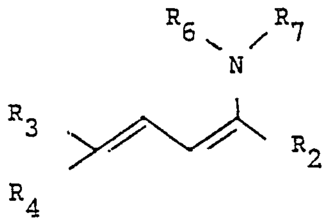

(II)

dans laquelle $R_2$, $R_3$ et $R_4$ ont les mêmes significations que dans la formule (I) de la revendication 1, $R_6$ et $R_7$ représentent des radicaux alkyle ou $R_6$ et $R_7$ forment ensemble avec l'atome d'azote auquel ils sont rattachés un hétérocycle à l'exception du diméthylamino-2 cyano-5 phényl-5 pentadiène-2,4 oate d'éthyle caractérisé en ce que l'on fait réagir un sel de vinamidinium de formule :

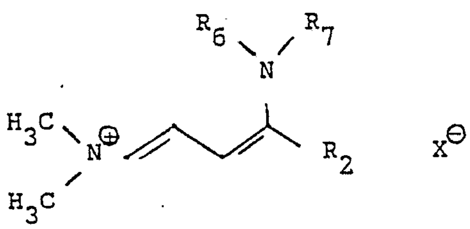

(III)

dans laquelle $R_2$, $R_6$ et $R_7$ ont les mêmes significations que précédemnent et X représente $BF_4$, Cl, $ClO_4$, Br sur un composé de formule :

$R_3 - CH_2 - R_4$

dans laquelle $R_3$ et $R_4$ ont les mêmes significations que précédemment puis isole le produit.

3 - Procédé de préparation des énamines de formule :

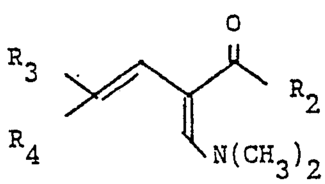

(VI)

dans laquelle $R_3$ et $R_4$ ont les mêmes significations que dans la formule (I) de la revendication 1 et $R_2$ représente un radical alcoxycarbonyle caractérisé en ce que l'on fait réagir un dialkylacétal de N,N-diméthylformamide sur un acide correspondant de formule (I) dans laquelle $R_2$ représente le radical carboxy, $R_1$ représente le radical hydroxy, $R_3$ et $R_4$ sont définis comme précédemment puis isole le produit.

Office européen des brevets

**RAPPORT PARTIEL DE RECHERCHE EUROPEENNE**
qui selon la règle 45 de la Convention sur le brevet européen est considéré, aux fins de la procédure ultérieure, comme le rapport de recherche européenne

Numéro de la demande
EP 88 40 2416

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | RECUEIL, JOURNAL OF THE ROYAL NETHERLANDS CHEMICAL SOCIETY, vol. 98, no. 2, février 1979, pages 36-41, La Haye, NL H.C.J.G. VAN BALEN et al.: " Chemistry of electron-rich conjugated polyenes III. Synthesis and acid hydrolysis of 2,2-di- and 2,2,5-trimethoxy-5,6-dihydropyrans" * Page 39, colonne de droite, avant-dernier paragraphe * | 1 | C 07 C 149/00<br>C 07 C 69/00<br>C 07 C 87/24<br>A 61 K 31/10 |

-------------

DOMAINES TECHNIQUES RECHERCHES (Int. Cl.4)

C 07 C 149/00
C 07 C 121/00
C 07 D 335/00
C 07 D 295/00
C 07 D 317/00

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.
Revendications ayant fait l'objet de recherches complètes:
Revendications ayant fait l'objet de recherches incomplètes:
Revendications n'ayant pas fait l'objet de recherches:
Raison pour la limitation de la recherche:

L'emploi en série de symboles représentant des substituents, ainsi que l'emploi repété d'alternatives et de conditions font que la demande ne satisfait pas nécessairement aux éxigences des articles 82 à 84 de la CBE (unité d'invention, clarté des revendications)

| Lieu de la recherche | Date d'achèvement de la recherche | Examinateur |
|---|---|---|
| La Haye | 06-12-1988 | VAN GEYT |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1505.1 03.82